# DEMANDE DE BREVET EUROPEEN

(11) **EP 1 889 599 A1**
(43) Date de publication de la demande: **20.02.2008**
(21) Numéro de dépôt: 07301258.5
(22) Date de dépôt: 20.07.2007
(51) Int. Cl.: A61K 8/19, A61K 8/25, A61Q 1/08

(54) **Composition et procédé de maquillage des peaux foncées**

(30) Priorité: 21.07.2006 FR 0606667; 21.07.2006 FR 0606669; 21.07.2006 FR 0606672; 21.07.2006 FR 0606665; 21.07.2006 FR 0606659; 21.07.2006 FR 0606661; 21.07.2006 FR 0606658; 21.07.2006 FR 0606664; 21.07.2006 FR 0606663
(71) Demandeur: L'ORÉAL, 75008 Paris (FR)
(72) Inventeur: Thevenet, Ludovic, 92340, BOURG LA REINE (FR); Chevalier, Gaëtan, 45760, BOIGNY SUR BIONNE (FR); Girier-Duffournier, Franck, 75011, PARIS (FR)
(74) Mandataire: Tanty, François

(57) **Abrégé**

La présente invention concerne un procédé de maquillage des peaux foncées, comportant l'étape consistant à appliquer sur la peau une composition cosmétique comportant dans un milieu cosmétiquement acceptable au moins un pigment interférentiel de couleur rouge, apte à générer, lorsque la composition est appliquée sur la peau, des points de surbrillance d'intensité supérieure ou égale à 3000 cd m⁻² et de longueur d'onde dominante comprise entre 580 nm et 650 nm.

## Description

La présente invention vise à proposer des compositions cosmétiques à appliquer sur la peau et plus particulièrement des compositions destinées aux peaux foncées, y compris noires et métissées.

Il existe un besoin pour bénéficier d'une composition de maquillage plus particulièrement destinée aux peaux foncées, qui permette de procurer à ce type de peau un résultat couleur éclatant.

La présente invention a ainsi pour objet, selon l'un de ses aspects, un procédé de maquillage des peaux foncées, comportant l'étape consistant à appliquer sur la peau une composition cosmétique comportant dans un milieu cosmétiquement acceptable au moins un pigment interférentiel de couleur rouge, apte à générer, lorsque la composition est appliquée sur la peau, des points de surbrillance d'intensité supérieure ou égale à 3 000 cd m⁻² et de longueur d'onde dominante comprise entre 580 nm et 650 nm.

L'invention permet d'obtenir un maquillage d'un plus bel effet sur les peaux foncées, notamment un maquillage créant la perception d'un teint réchauffé par cette couleur particulière et lumineux grâce à la brillance de ce pigment.

La composition peut présenter une saturation C* supérieure ou égale à 30, voire supérieure ou égale à 35.

La composition peut présenter un angle de teinte h° compris entre 10° et 30°, notamment entre 15° et 25°.

Les épaisseurs optiques (produit de l'épaisseur de la couche produisant l'interférence par l'indice de réfraction) du pigment interférentiel de couleur rouge peuvent aller de 310 nm à 430 nm pour l'ordre 1 d'interférence et aller de 620 nm à 860 nm pour l'ordre2. Ces épaisseurs optiques couvrent la couleur rouge (620 nm à 700 nm) pour deux ordres d'interférence en tenant compte d'une variation d'angle de 0 à 70), pour les milieux cosmétiques (indice de réfraction variant par exemple de 1,4 à 1,5).

La présente invention a également pour objet une composition de maquillage comportant, dans un milieu cosmétiquement acceptable, au moins un pigment interférentiel de couleur rouge, apte à générer, lorsque la composition est appliquée sur la peau, des points de surbrillance d'intensité supérieure ou égale à 3000 cd m⁻² et de longueur d'onde dominante comprise entre 580 nm et 650 nm et au moins un colorant.

Ce colorant peut avoir une longueur d'onde dominante comprise entre 580 et 650 nm, afin de créer un effet chatoyant lorsque la taille du pigment interférentiel rouge est de l'ordre du pouvoir séparateur de l'oeil.

Selon un autre de ses aspects, l'invention a pour objet une composition cosmétique comportant, dispersé dans un milieu cosmétiquement acceptable, un pigment interférentiel rouge apte à créer des points de surbrillance de longueur d'onde dominante comprise entre 580 et 650 nm et d'intensité supérieure ou égale à 3500 cd.m⁻², lorsque la composition est appliquée sur un support, la composition ne contenant pas de corps solides générant une couleur par absorption ou de charges blanches dans le milieu ou, lorsque la composition en contient, la teneur massique totale en de tels corps solides étant inférieure ou égale à 1 % par rapport au poids total de la composition.

Grâce à cet aspect de l'invention, la couleur produite par le phénomène d'interférences reste très nettement prépondérante par rapport à celle générée par absorption et un maquillage rouge éclatant peut être obtenu.

Selon cet aspect encore, la composition peut ne pas contenir de charges blanches ou de pigments diffusants dans le milieu.

De plus, la nature et la quantité des corps solides autres que le pigment interférentiel rouge pourront être fonction des propriétés optiques et des textures recherchées, à condition de ne pas entraver outre mesure le phénomène d'interférences responsable des points de surbrillance rouges.

Selon un autre de ses aspects, l'invention a pour objet une composition cosmétique comportant, dans un milieu cosmétiquement acceptable :
- au moins un premier pigment interférentiel apte à générer des points de surbrillance de couleur rouge d'intensité supérieure ou égale à 3 000 cd m⁻² et de longueur d'onde dominante comprise entre 580 et 650 nm, lorsque la composition est appliquée sur un support,
- des particules réfléchissantes aptes à générer sur ledit support d'autres points de surbrillance, d'intensité de brillance supérieure ou égale à celle du pigment interférentiel rouge, mieux supérieure ou égale à 4 000 cd m⁻².

Grâce à cet aspect, l'invention permet de modifier l'aspect de la composition sans affecter outre mesure la couleur rouge produite par le pigment interférentiel rouge.

Les particules réfléchissantes précitées peuvent notamment être utilisées en une teneur relativement faible tout en permettant, grâce à leur réflectivité, de modifier la clarté de la composition. De plus, ces particules réfléchissantes absorbent la lumière dans une moindre mesure que les pigments diffusants conventionnels générant une couleur par un phénomène d'absorption. Selon un autre de ses aspects, l'invention a pour objet une composition cosmétique comportant, dans un milieu cosmétiquement acceptable, un pigment interférentiel rouge apte à générer, lorsque la composition est appliquée sur un support, des points de surbrillance d'intensité supérieure ou égale à 3 000 cd m⁻² et de longueur d'onde dominante comprise entre 580 et 650 nm, présentant un indice de turbidité inférieur ou égal à 100 NTU.

Grâce à cet aspect de l'invention, la couleur produite par le phénomène d'interférences peut rester très nettement prépondérante par rapport à celle générée par absorption pour certaines conditions d'observation. Lorsque ces dernières changent, la couleur générée par absorption peut être perçue par l'observateur.

Selon un autre de ses aspects, l'invention a pour pour objet une gamme d'au moins deux compositions cosmétiques comportant, dispersé dans un milieu cosmétiquement acceptable, au moins un pigment interférentiel rouge apte à générer, lorsque la composition correspondante est appliquée sur un support, des points de surbrillance d'intensité supérieure ou égale à 3000 cdm⁻² et de longueur d'onde dominante comprise entre 580 et 650 mm, la différence de saturation entre deux compositions de la gamme étant inférieure ou égale à 2, ce pigment interférentiel rouge étant dans ces deux compositions à des concentrations différant d'au moins 1 %.

La gamme peut comporter plus de deux compositions et la relation ci-dessus peut être vérifiée, le cas échéant, pour deux compositions quelconque de la gamme ou pour certaines d'entre elles seulement.

Une telle gamme de compositions permet d'avoir des concentrations différentes en points de surbrillance rouge, et la demanderesse a constaté que, de manière inattendue, la présence d'un tel pigment interférentiel à des concentrations différentes n'entraînait pas une modification importante de la saturation.

Les compositions peuvent présenter sensiblement le même milieu.

Par « sensiblement le même milieu », il faut comprendre que les mêmes composés se retrouvent dans les compositions, à des concentrations pouvant varier en fonction de la teneur en pigment interférentiel rouge.

Ainsi, un composé peut être en une proportion différente d'une composition à l'autre afin de compenser la variation de la teneur en pigment interférentiel rouge.

Les compositions peuvent ne pas comporter d'autres corps solides que le pigment interférentiel rouge.

Les teneurs massiques en pigment interférentiel rouge entre les deux compositions précitées de la gamme peuvent différer d'au moins 2 %.

Dans tout ce qui suit, l'expression « la composition » peut désigner l'une quelconque des compositions de la gamme.

Selon un autre de ses aspects, l'invention a pour objet une composition cosmétique comportant, dans un milieu cosmétique acceptable :
- un pigment interférentiel de couleur rouge apte à générer des points de surbrillance d'intensité supérieure ou égale à 3000 cd.m⁻² et de longueur d'onde dominante comprise entre 580 et 650 mm,
- des corps magnétiques présentant une susceptibilité magnétique non nulle.

Selon cet aspect, l'invention exploite la sensibilité toute particulière du pigment interférentiel rouge à son environnement. Ainsi, grâce à la présence de ce pigment interférentiel, même une faible modification de l'orientation et/ou de l'emplacement des corps magnétiques dans la composition est susceptible de conduire dans l'invention à un effet visuellement observable, par exemple une variation de l'intensité et/ou de la concentration des points de surbrillance, grâce notamment à un masquage plus ou moins important du pigment interférentiel rouge par les corps magnétiques.

La composition peut prendre un état empêchant tout nouveau changement d'orientation des corps magnétiques sous l'effet d'un champ magnétique après une durée de séchage donnée. C'est par exemple le cas d'un vernis à ongles.

L'orientation des corps magnétiques peut encore, dans certains cas, être modifiée à tout moment, notamment lorsque la composition ne sèche pas ou présente une durée de séchage très longue. Cela peut être le cas par exemple d'un fond de teint.

Le champ magnétique est par exemple exercé peu de temps après le dépôt, de manière à changer l'aspect de la composition avant séchage de celle-ci, lorsque la composition comporte un solvant volatil.

Le cas échéant, les corps magnétiques peuvent être constitués par le pigment interférentiel rouge, lorsque celui-ci présente une susceptibilité magnétique non nulle.

Selon un autre de ses aspects, l'invention a encore pour objet une composition cosmétique comportant, dispersés dans un milieu cosmétique acceptable :
- un premier pigment interférentiel rouge apte à générer, lorsque la composition est appliquée sur un support, des points de surbrillance rouge d'intensité supérieure ou égale à 3 000 cd.m⁻² et de longueur d'onde dominante λ₁ comprise entre 580 et 650 nm,
- un deuxième pigment réfléchissant argenté ou un pigment réfléchissant coloré et de longueur d'onde dominante λ₂ telle que |λ₁ - λ₂| ≥ 50 nm, ce deuxième pigment ayant une taille moyenne supérieure ou égale à 30 µm, mieux à 40 µm.

Le deuxième pigment peut être un pigment interférentiel.

La demanderesse a constaté que le deuxième pigment peut apporter de nouveaux effets coloriels tout en permettant à la composition de conserver l'intensité de brillance du pigment interférentiel rouge, les premier et deuxième pigments pouvant créer, en quelque sorte, une mosaïque colorée.

Une difficulté peut se poser dans la formulation de la composition lorsque l'on cherche à avoir des intensités de surbrillance du même ordre pour le pigment interférentiel rouge et pour les pigments réfléchissants colorés, afin d'obtenir un effet de pixellisation relativement homogène en intensité.

Lorsque les pigments réfléchissants colorés présentent une structure multicouche, il peut être avantageux d'utiliser un pigment interférentiel rouge et des pigments réfléchissants colorés ayant un même coeur, car cela peut permettre d'obtenir plus facilement un même état de surface, lequel influe fortement sur l'intensité de brillance.

L'utilisation d'un même coeur peut également permettre d'obtenir plus facilement une même couleur générée par absorption lorsque le pigment interférentiel rouge et les pigments réfléchissants colorés présentent une couche de surface réalisée dans le même matériau, ce qui peut être intéressant pour que le pigment interférentiel rouge et les pigments réfléchissants colorés apparaissent de la même couleur sous éclairage rasant

Selon un autre de ses aspects, l'invention a pour objet une composition cosmétique comportant, dans un milieu cosmétiquement acceptable :
- au moins un pigment interférentiel rouge apte à générer, lorsque la composition est appliquée sur un support, des points de surbrillance d'intensité supérieure ou égale à 3000 cd m⁻² et de longueur d'onde dominante comprise entre 580 et 680 nm,
- au moins un agent de coloration sensible à au moins un stimulus extérieur.

L'utilisation combinée du pigment interférentiel rouge et de l'agent de coloration Xchrome permet d'obtenir au moins deux aspects différents pour la composition, selon l'état de l'agent de coloration Xchrome.

Il peut s'avérer notamment plaisant sur le plan esthétique que dans l'un de ses états, l'agent de coloration Xchrome prenne une couleur rouge car cela peut diminuer le contraste des points de surbrillance rouge et rendre ces derniers moins visibles. Le changement d'état de l'agent de coloration Xchrome s'accompagne alors d'une meilleure perception des points de surbrillance rouge et l'observateur peut être surpris de voir briller intensément le pigment interférentiel.

L'agent de coloration Xchrome peut encore, en changeant d'état, influer sur la diffusion de la lumière dans l'environnement du pigment interférentiel rouge en agissant comme un filtre coloré ou localement comme une source d'éclairage secondaire.

L'agent de coloration Xchrome peut être choisi dans un exemple de mise en oeuvre de l'invention de manière à prendre deux états au moins dans lesquels le phénomène d'interférences est affecté et ne l'est pas ou est affecté à des degrés différents.

L'agent de coloration sensible à un stimulus extérieur peut être en solution dans le milieu, ce qui peut être le cas par exemple pour un agent solvatochrome. Cela peut permettre d'éviter une diffusion de la lumière par l'agent Xchrome et d'affaiblir le phénomène d'interférences.

Il peut s'avérer particulièrement avantageux que le pigment interférentiel rouge présente une taille comprise entre 30 µm et 80 µm, soit sensiblement du même ordre que le pouvoir séparateur de l'oeil, plus préférentiellement autour de 40 µm et que l'agent de coloration Xchrome prenne dans un état une couleur rouge. On peut alors obtenir un fond rouge mat avec des points de surbrillance qui semblent scintiller du fait de leur taille particulière, ce qui crée un effet chatoyant.

Selon un autre de ses aspects, l'invention a pour objet un ensemble comportant :
- une première composition cosmétique à appliquer sur des matières kératiniques, comportant au moins un agent de coloration apte à générer une couleur par absorption ou une charge diffusante,
- une deuxième composition cosmétique à appliquer sur la première et comportant un milieu cosmétiquement acceptable, dans lequel est dispersé au moins un pigment interférentiel rouge apte à créer, lorsque la deuxième composition est appliquée sur un support, des points de surbrillance d'intensité supérieure ou égale à 3000 cd.m⁻² et de longueur d'onde dominante comprise entre 580 et 650 mm.

Grâce à cet aspect de l'invention, le phénomène d'interférences n'est pas gêné par la présence du pigment diffusant ou de la charge car celui-ci ou celle-ci est présent dans la couche de base sous-jacente et n'entrave par conséquent pas la propagation de la lumière dans la couche de recouvrement contenant le pigment interférentiel rouge.

De préférence, le milieu dans lequel le pigment interférentiel rouge est dispersé est transparent, ce qui permet de voir le dépôt sous-jacent.

Selon un autre de ses aspects, l'invention a également pour objet, indépendamment de ce qui précède, un ensemble comportant :
- une composition de base comportant un milieu cosmétiquement acceptable dans lequel est dispersé au moins un pigment interférentiel rouge apte à créer, lorsque la composition est appliquée sur un support, des points de surbrillance d'intensité supérieure ou égale à 3000 cd.m⁻² et de longueur d'onde dominante comprise entre 580 et 650 mm,
- une composition de recouvrement à appliquer sur celle-ci. Cette autre composition peut être transparente et peut permettre par exemple d'améliorer la brillance et de créer un effet de loupe sur les points de surbrillance rouge.

La composition de recouvrement peut comporter un milieu d'indice de réfraction supérieur à celui dans lequel est dispersé le pigment interférentiel rouge.

La première composition peut être destinée à former la couche de base et peut présenter toute formulation compatible avec le dépôt ultérieur de la deuxième composition.

La première composition peut notamment comporter un milieu cosmétiquement acceptable, tel que défini ci-après, et au moins un agent de coloration ou une charge diffusante.

La deuxième composition contient le pigment interférentiel rouge, dispersé dans un milieu cosmétiquement acceptable.

La deuxième composition est par exemple destinée à être appliquée sur la première.

### Peaux foncées

Au sens de la présente invention, on entend par "peaux foncées", des peaux dont la clarté moyenne L* mesurée sur le front, les pommettes et le menton dans l'espace colorimétrique CIE 1976, est inférieure à 55.

La saturation C* peut être comprise par exemple entre 10 et 30, notamment entre 12 et 28. Les valeurs d'angle de teinte h en ° peuvent être comprises par exemple entre 38° environ et 54° environ. Les valeurs de clarté L* peuvent être inférieures ou égales à 50, voire 45 ou 40 pour les peaux les plus sombres, tout en pouvant rester, pour la plupart des peaux, supérieures à 30.

Des peaux foncées sont rencontrées par exemple parmi les populations africaine, afro-américaine, hispano-américaine, indienne et maghrébine.

Ces peaux peuvent encore être classées sur la base de leur réactivité aux effets du rayonnement solaire selon l'échelle proposée par FITZPATRICK.

Selon cette échelle, les différentes peaux ethniques existantes peuvent être distinguées selon les types suivants :

| **Type** | **Réactivité de la peau** | **Origine** |
|---|---|---|
| I | Brûle toujours, ne bronze jamais | Celte |
| II | Brûle toujours, bronze peu | Germanique |
| III | Brûle modérément, bronze progressivement | Européen |
| IV | Brûle faiblement, bronze très facilement | Méditerranéen |
| V | Brûle rarement, bronze intensément | Moyen Orient - Sud Américain |
| VI | Ne brûle jamais, fortement pigmentée | Africain |

Les peaux foncées qui font plus particulièrement l'objet de la présente invention appartiennent aux types IV à VI.

### Mesure de la saturation

La composition est étalée sur une carte de contraste de marque LENETA avec une épaisseur de 300 µm. La mesure est effectuée sur le fond noir de la carte.

La réflectance est mesurée à l'aide du spectrocolorimètre Minolta 3700-d (d65/10°) en mode composante spéculaire exclue, petite ouverture (CREISS).

Les spectres de réflectance ainsi acquis sont exprimés en coordonnées colorimétriques dans l'espace CIELab76 au sens de la Commission Internationale de l'Eclairage selon la recommandation 15 :2004.

### Mesure de l'intensité des points de surbrillance

Pour mesurer l'intensité des points de surbrillance, la composition étudiée est étalée sur une carte de contraste, par exemple de marque LENETA, sur une épaisseur de 300 µm.

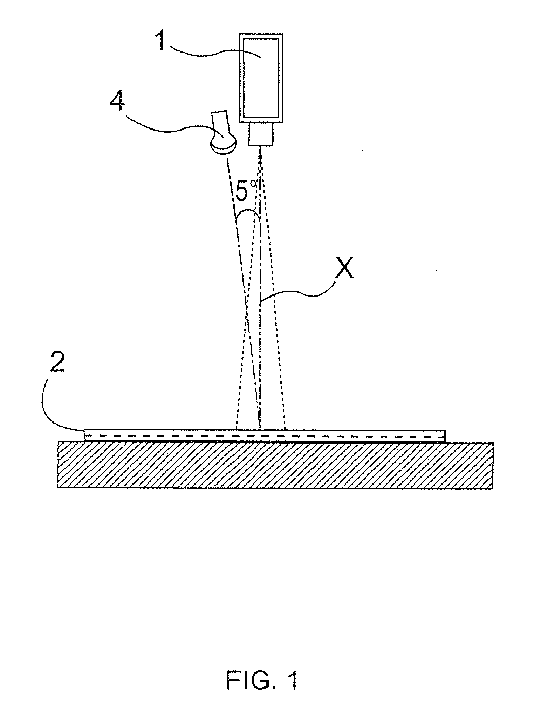

La composition ainsi étalée est placée devant une caméra colorimétrique 1 selon l'agencement représenté à la **figure 1**. Sur cette figure, on voit que la carte de contraste 2 revêtue de la composition est placée perpendiculairement à l'axe optique X de la caméra 1 et que l'éclairage s'effectue au moyen d'une source lumineuse 4 (illuminant D65) émettant dans une direction faisant un angle de 5° avec l'axe optique X.

La surbrillance est définie comme étant l'intensité lumineuse émise de façon localisée.

La caméra présente une résolution dans le plan xy de quelques µm, suffisante pour différencier très nettement les différentes particules présentes dans la composition.

Le système optique est par exemple le photomètre et le colorimètre imageant LUMICAM 1300 de la société INSTRUMENT SYSTEM.

Les mesures de luminance peuvent être réalisées dans la gamme 0,2 à 200 000 cd.m⁻² avec une fidélité de mesure de 4%, une répétabilité de 0,1 % et une uniformité de 1,5 % (pour une zone de 10 x 10 pixels).

Le système optique comporte un objectif macro 105 mm ayant un angle de champ de 5°, une focale de 22 mm, placé à une distance de 48 cm de la composition. La zone de mesure s'étend sur 2,9 x 2,7 mm.

La sensibilité est de 100 iso, la vitesse d'obturation de 1/60s et l'ouverture de f:2.

Le dispositif expérimental illustré permet d'éliminer la réflexion spéculaire sur la surface du film de la composition.

Le résultat obtenu est sous forme de matrice bidimensionnelle où chaque élément M_{i,j} représente l'intensité détectée par la cellule de coordonnées i,j dans le plan x y, en candela par m², 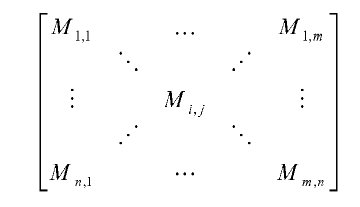 où :
m désigne le nombre de pixels dans la direction x du système de détection, et
n désigne le nombre de pixels dans la direction y du système de détection.

La longueur d'onde dominante est mesurée avec le colorimètre imageant.

### Mesure de la turbidité

La turbidité correspond à la réduction de la transparence d'un liquide due à la présence de particules en suspension, et se mesure en faisant passer un faisceau lumineux à travers l'échantillon à tester

La turbidité peut dépendre de l'indice de réfraction du milieu et de la nature et de la concentration des corps en suspension dans celui-ci.

L'indice de turbidité est déterminé en mesurant la lumière qui est diffusée par les particules en suspension, au moyen d'un turbidimètre, en l'espèce celui de référence 2100 P de la société HACH.

### Mesure du trajet de couleur

Lorsque la composition présente un indice de turbidité inférieur ou égal à 100 NTU, elle permet d'obtenir un trajet de couleur relativement important, car la faible teneur totale en particules en suspension ne gêne pas l'observation de la couleur produite par absorption par la couche de surface du pigment interférentiel rouge sous forte incidence.

Le « trajet de couleur » désigne une variation dans le plan a*b* de l'espace colorimétrique CIE 1976 et peut être mesuré par exemple au moyen d'un spectrogonioréflectomètre de marque INSTRUMENT SYSTEMS et de référence GON 360 GONIOMETER, après que la composition ait été étalée à l'état fluide avec une épaisseur de 300 µm au moyen d'une étaleur automatique sur une carte de contraste de marque ERICHSEN et de référence Typ 24/5, la mesure étant effectuée sur le fond noir de la carte.

Le trajet de couleur d'une composition selon l'invention correspond par exemple à une variation Dh de l'angle de teinte h d'au moins 20° lorsque l'on fait varier l'angle d'observation par rapport à la normale entre 0° et 80°, pour un angle d'incidence de la lumière de 45°.

### Pigment interférentiel rouge

Ce pigment est apte, selon l'invention, à générer des points de surbrillance de longueur d'onde dominante comprise entre 580 nm et 650 nm, mieux 580 nm et 600 nm, et d'intensité supérieure ou égale à 3 000 cd.m⁻², mieux 3 400 cd.m², encore mieux 4 200 cd m⁻². L'intensité peut être inférieure à 5 000 cd m⁻².

La teneur en pigment interférentiel rouge peut aller par exemple de 0,1 % à 15 % si le milieu est liquide et de 0,1 à 60 % si le milieu est solide.

De préférence, la taille de ce pigment, définie par la distribution granulométrique moyenne à la moitié de la population, encore appelée D₅₀, est supérieure ou égale à 30 µm, mieux 40 µm, par exemple comprise entre 30 et 80 µm, mieux 30 et 70 µm.

Le pigment présente avantageusement une forme générale aplatie, son épaisseur étant par exemple inférieure ou égale à 5 µm, de préférence inférieure ou égale à 3 µm

La structure multicouche peut être symétrique ou non, étant de préférence symétrique.

Le pigment peut comporter un coeur d'un matériau organique ou inorganique, recouvert d'une ou plusieurs couches de matériaux organiques ou inorganiques.

Le pigment peut comporter par exemple un coeur de silice, de mica ou de verre, enrobé par une couche d'oxyde de fer Fe₂O₃ ou d'un autre oxyde métallique, par exemple un oxyde de titane ou d'étain.

L'épaisseur de la ou des différentes couches recouvrant le coeur sera déterminée par la théorie de la réflexion de la lumière sur les couches minces, de manière à ce que la lumière réfléchie ait la longueur d'onde dominante recherchée.

De préférence, le coeur est de forme générale aplatie et le pigment présente des faces principales sensiblement planes, de façon à permettre une réflexion spéculaire intense.

Le pigment peut, le cas échéant, présenter une susceptibilité magnétique non nulle.

Comme exemple de pigment interférentiel rouge disponible commercialement, on peut citer celui commercialisé sous la référence XIRONA LE ROUGE par la société MERCK.

### Milieu cosmétiquement acceptable

Par « milieu cosmétiquement acceptable », on désigne un milieu non toxique susceptible d'être appliqué sur les matières kératiniques d'êtres humains.

Le milieu cosmétiquement acceptable sera adapté à la nature du support sur lequel doit être appliqué la composition ainsi qu'à la forme sous laquelle la composition est destinée à être conditionnée.

La composition selon l'invention peut comprendre un milieu aqueux et/ou une phase grasse.

### Phase aqueuse ou grasse

La composition peut comprendre de l'eau ou un mélange d'eau et de solvants organiques hydrophiles comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols.

La phase hydrophile peut, en outre, contenir des éthers en C₂ et des aldéhydes en C₂-C₄ hydrophiles.

L'eau ou le mélange d'eau et de solvants organiques hydrophiles peut être présent dans la composition selon l'invention en une teneur allant de 0 % à 90 %, notamment 0,1 % à 90 % en poids, par rapport au poids total de la composition, et de préférence de 0 % à 60 % en poids, notamment 0,1 % à 60 % en poids.

La composition peut également comprendre une phase grasse, notamment constituée de corps gras liquides à 25°C et éventuellement de corps gras solides à température ambiante tels que les cires, les corps gras pâteux, les gommes et leurs mélanges.

Comme corps gras liquides à température ambiante, appelés souvent huiles, utilisables dans l'invention, on peut citer entre autres les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et, notamment les isoparaffines en C₈-C₁₆ telles que l'isododécane, l'isodécane, l'isohexadécane, la vaseline, les polydécènes, le polyisobutène hydrogéné tel que le Parléam®, le squalane; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent être présentes en une teneur allant de 0,01 à 90 % par rapport au poids total de la composition.

La composition selon l'invention peut également comprendre un ou plusieurs solvants organiques, physiologiquement acceptables. Ce ou ces solvants, qui peuvent être lipophiles, peuvent être présents en une teneur allant de 0 à 90 %, mieux de 0 à 60% en poids, par rapport au poids total de la composition et encore mieux de 0,1 à 30 %.

Le milieu peut comporter une phase organique liquide dans laquelle de l'eau est dispersée ou émulsionnée.

La composition peut encore présenter une phase grasse continue, pouvant contenir moins de 5% d'eau, notamment moins de 1 % d'eau par rapport à son poids total et en particulier être sous forme anhydre.

### Agent filmogène

Le milieu peut comporter un agent filmogène, notamment un polymère filmogène.

On entend par « agent filmogène » un agent apte à former à lui seul ou en présence d'un agent auxiliaire de filmification, un film macroscopiquement continu et adhérent sur les matières kératiniques, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconnée.

La composition peut comporter une phase aqueuse et le polymère filmogène peut être présent dans cette phase aqueuse. Celui-ci pourra être un polymère en dispersion ou en solution.

La composition peut comporter une phase huileuse et le polymère filmogène peut être présent dans cette phase huileuse. Le polymère pourra alors être en dispersion ou en solution.

Parmi les polymères filmogènes utilisables, on peut citer les polymères synthétiques, de type radicalaire ou de type polycondensat, les polymères d'origine naturelle, et leurs mélanges.

Les polymères filmogènes de type radicalaire peuvent être notamment des polymères, ou des copolymères, vinyliques, notamment des polymères acryliques.

Parmi les polycondensats filmogènes, on peut citer les polyuréthanes, les polyesters, les polyesters amides, les polyamides, et les résines époxyesters, les polyurées.

Les polyesters peuvent être obtenus, de façon connue, par polycondensation d'acides dicarboxyliques avec des polyols, notamment des diols.

Les polyesters amides peuvent être obtenus de manière analogue aux polyesters, par polycondensation de diacides avec des diamines ou des amino alcools.

A titre d'exemple de polymères filmogènes liposolubles, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Le polymère filmogène peut encore être choisi parmi les résines de silicone, généralement solubles ou gonflables dans les huiles de silicone, qui sont des polymères de polyorganosiloxanes réticulés.

Le polymère filmogène peut être également présent dans la composition sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

Bien entendu, cette liste de polymères n'est pas exhaustive.

### Charges et autres agents de coloration

La composition cosmétique peut comprendre des charges, par exemple des charges incolores dans le milieu.

Par « charges », on désigne des particules de toute forme insolubles dans le milieu de la composition, quelle que soit la température à laquelle la composition est fabriquée. Ces charges peuvent servir notamment à modifier la rhéologie ou la texture de la composition.

A titre d'exemple de charges, on peut citer, entre autres, le talc, le mica, la silice, le kaolin, et les poudres de polyamide (Nylon^{®} ou Orgasol^{®} de chez Atochem).

Dans des exemples de mise en oeuvre de l'invention, les charges peuvent être blanches ou incolores dans le milieu. De préférence, on utilise des charges incolores dans le milieu plutôt que des charges blanches dans le milieu.

A titre d'exemple de charges incolores dans le milieu, on peut citer, entre autres, le mica et les poudres de matières thermoplastiques, les poudres de polyamide (par exemple Nylon^{®} ou Orgasol^{®} de chez Atochem), les poudres de PET, PE, PP, PVC, PMMA, PC.

A titre de charges blanches dans le milieu, on peut citer, entre autres, le talc, le dioxyde de titane, le sulfate de baryum, le kaolin, la silice et le sulfate de magnésium.

La teneur en charges sera choisie de façon à ne pas entraver outre mesure le phénomène d'interférences responsable des points de surbrillance rouges.

La composition peut comporter un agent de coloration autre le pigment interférentiel rouge, dans une teneur n'empêchant pas le phénomène d'interférences produisant les points de surbrillance rouges.

Cet agent de coloration peut être un pigment diffusant apte à générer une couleur par un phénomène d'absorption, comportant une matière colorante organique ou inorganique.

L'agent de coloration peut encore comporter un pigment diffractant, une nacre ou un pigment goniochromatique.

L'agent de coloration peut encore comporter un colorant.

Il peut s'agir d'un colorant d'origine végétale, animale ou minérale, en particulier d'origine végétale ou minérale, notamment d'origine végétale. Ce colorant peut être de nature non synthétique.

Le colorant peut être un colorant naturel hydrosoluble ou liposoluble.

A titre illustratif des agents de coloration naturels hydrosolubles susceptibles d'être mis en oeuvre selon l'invention, on peut particulièrement citer le caramel, le jus de betterave et le carmin, la bétanine (betterave), la chlorophylline cuivrée, le bleu de méthylène, les anthocyanines (enocianine, carotte noire, hibiscus, sureau) et la riboflavine.

A titre illustratif des agents de coloration naturels liposolubles susceptibles d'être mis en oeuvre selon l'invention, on peut particulièrement citer le rouge Soudan, le β-carotène, les caroténoïdes, le lycopène, l'huile de palme, le brun Soudan, le jaune quinoléique les xanthophylles (capsanthine, capsorubine, lutéine), et le curcumin.

Comme autres colorants naturels, on peut plus particulièrement citer les anthocyanes de fleurs ou de fruits ou leurs dérivés, les flavonoïdes et tanins extraits de végétaux natifs ou fermentés, la juglone, la lawsone, les extraits de soja fermenté, d'algues, de champignons, de micro-organismes, les sels de Flavylium non substitués en position 3 tels que décrit dans le brevet EP 1 172 091, les extraits de Gesneria Fulgens, Blechum Procerum, Saxifraga et les pigments susceptibles d'être obtenus par extraction par un solvant organique ou hydro-organique d'un milieu de culture de micromycètes du type monascus Monascus.

Comme exemple de colorants synthétiques, on peut citer les colorants liposolubles synthétiques tels que, par exemple, le DC Red 17, le DC Red 21, le DC Red 27, le DC Green 6, le DC Yellow 11, le DC Violet 2 et le DC Orange 5.

Comme exemple de colorants hydrosolubles synthétiques, on peut citer le FDC Red 4, le DC Red 6, le DC Red 22, le DC Red 28, le DC Red 30, le DC Red 33, le DC Orange 4, le DC Yellow 5, le DC Yellow 6, le DC Yellow 8, le FDC Green 3, le DC Green 5 et le FDC Blue 1.

### Particules réfléchissantes

Diverses particules réfléchissantes à reflet métallique peuvent être envisagées, notamment celles présentant une réflectivité suffisamment élevée pour créer des points de surbrillance d'intensité supérieure ou égale à 3 000 cd m⁻², mieux à 4 000 cd m⁻² et par exemple inférieure ou égale à 5 000 cd m⁻².

Le ration m₁/m₂ entre la teneur massique m₁ en pigment interférentiel rouge et la teneur m₂ en particules réfléchissantes peut aller de 0,1 à 1,5.

Leur taille peut aller par exemple de 10 µm à 500 µm, étant de préférence comprise entre 10 et 150 µm. La taille peut avantageusement être supérieure ou égale à 40 µm.

Les particules réfléchissantes peuvent présenter une forme plaquettaire, ce qui peut rendre la réflexion plus directionnelle, ou au contraire une forme sensiblement sphérique, afin d'apporter une réflexion plus diffuse.

Les particules réfléchissantes sont par exemple à reflet métallique et comportent avantageusement au moins une couche conductrice de l'électricité en surface, formée d'au moins un métal ou oxyde métallique.

Les particules réfléchissantes à reflet métallique, quelle que soit leur forme, peuvent présenter une structure multicouche ou non et, dans le cas d'une structure multicouche, comporter par exemple au moins une couche ayant de préférence une épaisseur uniforme, notamment d'un matériau réfléchissant, avantageusement un composé métallique.

Lorsque les particules réfléchissantes à reflet métallique ne présentent pas de structure multicouche, elles peuvent être composées par exemple d'au moins un composé métallique, par exemple un oxyde métallique, notamment un oxyde de fer obtenu par synthèse.

Lorsque les particules réfléchissantes présentent une structure multicouche, celles-ci peuvent par exemple comporter un substrat naturel ou synthétique, notamment un substrat synthétique au moins partiellement enrobé par au moins une couche d'un matériau réfléchissant, notamment au moins une couche d'au moins un composé métallique tel qu'un métal ou alliage. Le substrat peut être monomatière, multimatériau, organique et/ou inorganique. Plus particulièrement, le substrat peut être choisi parmi les verres, les céramiques, le graphite, les oxydes métalliques, les alumines, les silices, les silicates, notamment les aluminosilicates et les borosilicates, le mica synthétique et leurs mélanges, cette liste n'étant pas limitative.

A titre d'exemple de particules réfléchissantes comportant un substrat minéral enrobé d'une couche de métal, on peut citer les particules comportant un substrat de borosilicate enrobé d'argent. Des particules à substrat de verre revêtu d'argent, en forme de plaquettes, sont vendues sous la dénomination de MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société.

Les particules réfléchissantes à reflet métallique, quelle que soit leur forme, peuvent également être choisies parmi les particules à substrat synthétique enrobé au moins partiellement d'au moins une couche d'au moins un oxyde métallique, choisi par exemple parmi les oxydes de titane, notamment Ti0₂, de fer notamment Fe₂0₃, d'étain, de chrome, le sulfate de baryum et les matériaux suivants : MgF₂, CrF₃, ZnS, ZnSe, SiO₂, AI₂0₃, Mg0, Y₂0₃, Se0₃, Si0, Hf0₂, Zr0₂, Ce0₂, Nb₂0₅, Ta₂0₅, MoS₂ et leurs mélanges.

A titre d'exemple de telles particules, on peut citer par exemple les particules comportant un substrat de mica synthétique revêtu de dioxyde de titane, ou les particules de verre enrobé soit d'oxyde de fer brun, d'oxyde de titane, d'oxyde d'étain ou d'un de leurs mélanges comme celles vendues sous la marque REFLECKS® par la société ENGELHARD.

Comme autres exemples de particules réfléchissantes à reflet métallique présentant en surface un composé métallique ou incluant au moins un composé métallique enrobé, on peut citer les particules proposées sous les dénominations METASHINE® ME 2040 PS, METASHINE® MC5090 PS ou METASHINE® MC280GP (2523) par la société NIPPON SHEET GLASS, SPHERICAL SILVER POWDER® DC 100, SILVER FLAKE® JV6 ou GOLD POWDER® A1570 par la société ENGELHARD, STARLIGHT REFLECTIONS FXM® par la société ENERGY STRATEGY ASSOCIATES INC. BRIGHT SILVER® 1 E 0.008X0.008 par la société MEADOWBROOK INVENTIONS, ULTRAMIN® (ALUMINIUM POUDRE FINE LIVING), et COSMETIC METALLIC POWDER VISIONAIRE BRIGHT SILVER SEA®, COSMETIC METALLIC POWDER VISIONAIRE NATURAL GOLD® (60314) ou COSMETIC METALLIC POWDER VISIONAIRE HONEY® (60316) par la société ECKART.

Les particules réfléchissantes à reflet métallique peuvent réfléchir le spectre visible de manière sensiblement uniforme, comme c'est le cas par exemple de particules revêtues d'un métal tel que l'argent ou l'aluminium, ou non, ce qui peut alors conduire par exemple à une reflet métallique ayant un ton non neutre, jaune, rose, rouge, bronze, orangé, brun, or et/ou cuivré, selon la nature par exemple du composé métallique en surface.

Les particules réfléchissantes à reflet métallique peuvent être présentes dans la composition à une teneur allant de 0,1 % à 60 % par rapport au poids total de la première composition, notamment de 1 % à 30 % en poids, par exemple de 3 % à 10 % en poids.

Lorsque les particules réfléchissantes présentent une structure multicouche avec un coeur, ce coeur peut être dans le même matériau que celui du pigment interférentiel rouge.

### Pigment réfléchissant argenté

Ce pigment réfléchit le spectre de lumière incidente de manière sensiblement uniforme.

A titre d'exemples de pigments réfléchissants argentés, on peut citer les particules réfléchissantes argentées TIMICA SPARKLE 110P^{®}, TIMICA SILKBLANC 110W^{®}, FLAMENCO SUPERPEARL 120 C+^{®}, TIMICA EXTRA LARGE SPARKLE 110S^{®}, FLAMENCO PEARL 110C^{®}, TIMICA PEARL WHITE 110 A^{®}, TIMICA SILVER SPARKLE 5500 / EP 94003^{®}, FLAMENCO SATIN PEARL 3500^{®} commercialisées par la société ENGELHARD, les particules réfléchissantes argentées NAILSYN PLATINUM 60^{®}, XIRONA SILVER^{®}, BIRON LF 2000^{®} (ref 117077), TIMIRON SNOWFLAKE MP 99^{®} (117470), LOW LUSTRE PIGMENT^{®} (17399), TIMIRON DIAMOND CLUSTER MP 149^{®} (17266), TIMIRON ULTRALUSTER MP 111^{®} (117226), TIMIRON PEARL SHEEN MP 30^{®} (17216), TIMIRON SUPER SILK MP 1005^{®} (17203) commercialisées par la société MERCK, les particules réfléchissantes argentées PRESTIGE SPARKLING SILVER^{®} (35178), PRESTIGE SPARKLING SILVER STAR^{®} (35179) commercialisées par la société ECKART, les particules réfléchissantes argentées SUNSHINE FINE WHITE^{®} (C80-3100), SHUNSHINE GLITTER WHITE^{®} (C80-3400) commercialisées par la société SUN et les particules réfléchissantes argentées KTZ CLASSIC WHITE^{®} (10-40 MICRONS), KTZ STELLAR WHITE^{®} (20-80 MICRONS) commercialisées par la société TAIZHU.

### Pigments réfléchissants colorés

Divers pigments réfléchissants colorés autres que le pigment interférentiel rouge peuvent être envisagés, à condition de présenter une réflectivité suffisamment élevée pour créer des points de surbrillance d'intensité supérieure ou égale à 3 000 cd.m⁻², mieux à 4 000 cd.m⁻² et par exemple inférieure ou égale à 5 000 cd.m⁻².

Leur taille est de préférence supérieure ou égale à 30 µm, mieux à 40 µm, étant avantageusement du même ordre que celle du pigment interférentiel rouge, à 10 % près, afin d'obtenir un effet de pixellisation plus homogène. La taille peut notamment aller de 30 µm à 80 µm, par exemple.

Le pigment réfléchissant coloré peut présenter une longueur d'onde dominante différente de celle du pigment interférentiel rouge, par exemple inférieure ou égale à 580 nm, mesurée avec le colorimètre précité, dans les conditions de mesure de l'intensité des points de surbrillance.

Il peut être avantageux que le pigment réfléchissant coloré présente un coeur dans le même matériau que le pigment interférentiel rouge, car cela peut permettre d'avoir des intensités de surbrillance du même ordre, à 10 % près.

L'expression « du même ordre, à 10 % près » signifie que la taille ou l'intensité de surbrillance du pigment réfléchissant est comprise entre 0,9 et 1,1 fois celle du pigment interférentiel rouge.

La couche de surface du pigment réfléchissant coloré peut être dans le même matériau que celle du pigment interférentiel rouge, notamment lorsque le coeur est également dans le même matériau, les pigments différant alors par exemple par l'épaisseur de la couche de surface qui permet de générer une autre couleur par le phénomène d'interférences.

La proportion du pigment réfléchissant coloré est par exemple comprise ente 0,1 et 10 fois celle du pigment interférentiel rouge.

Des proportions voisines à 10 % près peuvent permettre l'obtention d'un effet homogène.

Les pigments réfléchissants colorés peuvent être choisis parmi les nacres et les pigments interférentiels goniochromatiques, entre autres.

Par « nacre », il faut comprendre des particules colorées de toute forme, irisées ou non, notamment produites par certains mollusques dans leur coquille ou bien synthétisées et qui présentent un effet de couleur par un phénomène d'interférences.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être introduites dans la composition, on peut notamment citer les pigments colorés TIMICA SPARKLE GOLD^{®}, CLOISONNE SPARKLE ROUGE 450J^{®}, FLAMENCO SPARKLE GOLD 220J^{®}, FLAMENCO SPARKLE GREEN 820J^{®}, FLAMENCO SPARKLE ORANGE 320J^{®}, FLAMENCO SPARKLE BLUE 620J^{®}, CLOISONNE SPARKLE GOLD 222J^{®}, CLOISONNE SPARKLE GOLD 222J^{®}, CLOISONNE SPARKLE BLUE-ROUGE 650J^{®}, FLAMENCO SPARKLE VIOLET 520J^{®}, CLOISONNE SPARKLE COPPER 350J^{®}, CLOISONNE SPARKLE BRONZE 250J^{®}, DUOCROME SPARKLE BY 226J^{®}, DUOCROME SPARKLE RY 224J / EP 98001^{®}, DUOCROME SPARKLE BR 426J^{®}, DUOCROME SPARKLE RB 624J / EP 98002^{®}, FLAMENCO SPARKLE RED 420J^{®} commercialisés par la société ENGELHARD, les pigments colorés TIMIRON DIAMOND CLUSTER MP 149 (17266) ^{®} commercialisés par la société MERCK et les pigments colorés KTZ ULTRA SHIMMER^{®} commercialisés par la société TAIZHU.

### Actifs et autres composés

La composition cosmétique peut également contenir un ou plusieurs actifs cosmétiques, dermatologiques, hygiéniques ou pharmaceutiques.

Comme actifs cosmétiques, dermatologiques, hygiéniques ou pharmaceutiques, utilisables dans les compositions de l'invention, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, antirides...). Ces actifs peuvent être utilisés par exemple à des concentrations de 0,001 à 15 % par rapport au poids total de la composition.

La composition cosmétique peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants les filtres UV, ou leurs mélanges.

La composition cosmétique peut, de plus, comprendre, selon le type d'application envisagée, les constituants classiquement utilisés dans les domaines considérés, qui sont présents en une quantité appropriée à la forme galénique souhaitée.

### Solvant volatil

La composition peut comporter au moins un solvant aqueux ou organique, notamment un solvant volatil organique.

Au sens de la présente invention, on entend par « solvant volatil » un solvant, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm Hg), et de préférence allant de 1,3 Pa à 13 000 Pa (0,01 à 100 mm Hg), et préférentiellement allant de 1,3 Pa à 1300 Pa (0,01 à 10 mm Hg).

La première composition peut comporter au moins un solvant volatil constitué par une huile volatile.

L'huile peut être une huile siliconée ou une huile hydrocarbonée, ou comporter un mélange de telles huiles.

Au sens de la présente invention, on entend par « huile siliconée », une huile comprenant au moins un atome de silicium, et notamment au moins un groupe Si-O.

On entend par « huile hydrocarbonée », une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ (appelées aussi isoparaffines) comme l'isododécane (encore appelé 2,2,4,4,6-pentaméthylheptane), l'isodécane, l'isohexadécane, et par exemple les huiles vendues sous les noms commerciaux d'ISOPARS^{®} ou de PERMETHYLS^{®}.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également citer les huiles linéaires alkyltrisiloxanes volatiles de formule générale (I) : 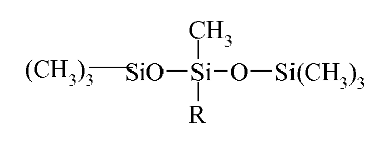 où R représente un groupe alkyl comprenant de 2 à 4 atomes de carbone et dont un ou plusieurs atomes d'hydrogène peuvent être substitués par un atome de fluor ou de chlore.

Parmi les huiles de formule générale (I), on peut citer :
- le 3-butyl 1,1,1,3,5,5,5-heptaméthyl trisiloxane,
- le 3-propyl 1,1,1,3,5,5,5- heptaméthyl trisiloxane, et
- le 3-éthyl 1,1,1,3,5,5,5-heptaméthyl trisiloxane,
correspondant aux huiles de formule (I) pour lesquelles R est respectivement un groupe butyle, un groupe propyle ou un groupe éthyle.

On peut également utiliser des huiles volatiles fluorées tels que le nonafluorométhoxybutane ou le perfluorométhylcyclopentane, et leurs mélanges.

Une composition selon l'invention peut comprendre par exemple entre 0,01 % et 95 % en poids d'huile volatile, par rapport au poids total de la composition, mieux entre 1 % et 75 % en poids.

La composition peut comporter au moins un solvant organique choisi dans la liste suivante :
- les cétones liquides à température ambiante, tels que le méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courter (ayant de 3 à 8 atomes de carbone au total) tels que l'acétate d'éthyle, l'acétate de méthyle, l'acétate de propyle, l'acétate de n-butyle, l'acétate d'isopentyle ;
- les alcanes liquides à température ambiante tels que le décane, l'heptane, le dodécane, le cyclohexane ;

La composition peut aussi comprendre de l'eau ou un mélange d'eau et de solvants organiques hydrophiles couramment utilisés en cosmétique comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols. La première composition peut, en outre, contenir des éthers en C₂ et des aldéhydes en C₂-C₄ hydrophiles. L'eau ou le mélange d'eau et de solvants organiques hydrophiles peut être présent dans la première et/ou la deuxième composition en une teneur allant par exemple de 0 % à 90 %, notamment de 0,1 % à 90 % en poids et de préférence de 0 % à 60 % en poids, notamment 0,1 % à 60 % en poids, par rapport au poids total de la composition.

### Corps magnétiques

L'expression « corps magnétiques » ne doit pas être comprise de manière limitative et couvre des particules, fibres ou agglomérats de particules et/ou de fibres, de toutes formes, présentant une susceptibilité magnétique non nulle.

La concentration en corps magnétiques dans la composition est choisie de manière à permettre au phénomène d'interférences de se manifester pour créer des points de surbrillance rouge. La concentration est par exemple comprise entre environ 0,05 % et environ 50 % en masse, notamment entre environ 0,1 % et environ 40 % en masse, mieux entre environ 1 % et environ 30 % en masse, selon la nature des corps magnétiques et leur incidence sur la diffusion de la lumière.

La composition appliquée peut comporter des fibres magnétiques ou autres corps asphériques, tels que des chaînes de particules ou de fibres.

De préférence, les corps magnétiques ne présentent pas d'aimantation rémanente en l'absence de champ magnétique.

Les corps magnétiques peuvent comporter tout matériau magnétique présentant une sensibilité aux lignes d'un champ magnétique, que ce champ soit produit par un aimant permanent ou issu d'une induction, ce matériau étant par exemple choisi parmi le nickel, le cobalt, le fer, leurs alliages et oxydes, notamment Fe₃O₄, et aussi le gadolinium, le terbium, le dysprosium, l'erbium, leurs alliages et oxydes. Le matériau magnétique peut être de type « doux » ou « dur ». Le matériau magnétique peut notamment être du fer doux.

Les corps magnétiques peuvent présenter ou non une structure multicouche, comportant au moins une couche d'un matériau magnétique, tel que par exemple le fer, le nickel, le cobalt, leurs alliages et oxydes, notamment Fe₃O₄.

Les corps magnétiques sont de préférence asphériques, présentant par exemple une forme allongée. Ainsi, lorsque ces corps sont soumis au champ magnétique, ils tendent à s'orienter avec leur axe longitudinal dans l'alignement des lignes de champ, et subissent un changement d'orientation qui se traduit par un changement d'aspect de la composition.

Lorsque les corps magnétiques sont des particules sensiblement sphériques, de préférence leur aspect est inhomogène, de manière à ce qu'un changement d'orientation induise un changement d'aspect.

La dimension des corps, quelle que soit leur forme, est par exemple comprise entre 1 nm et 10 mm, mieux entre 10 nm et 5 mm, mieux encore entre 100 nm et 1 mm, par exemple entre 0,5 µm et 300 µm ou 1 µm et 150 µm.

Lorsque les corps sont des particules n'ayant pas une forme allongée ou ayant une forme allongée avec un facteur de forme assez faible, la dimension des particules est par exemple inférieure à 1 mm.

Les corps magnétiques sont par exemple des pigments magnétiques.

### Pigments magnétiques

Des pigments convenant tout particulièrement sont les nacres comportant de l'oxyde de fer Fe₃O₄. Des pigments présentant des propriétés magnétiques sont par exemple ceux commercialisés sous les dénominations commerciales COLORONA BLACKSTAR BLUE, COLORONA BLACKSTAR GREEN, COLORONA BLACKSTAR GOLD, COLORONA BLACKSTAR RED, CLOISONNE NU ANTIQUE SUPER GREEN, MICRONA MATTE BLACK (17437), MICA BLACK (17260), COLORONA PATINA SILVER (17289) et COLORONA PATINA GOLD (117288) de la société MERCK ou bien encore FLAMENCO TWILIGHT RED, FLAMENCO TWILIGHT GREEN, FLAMENCO TWILIGHT GOLD, FLAMENCO TWILIGHT BLUE, TIMICA NU ANTIQUE SILVER 110 AB, TIMICA NU ANTIQUE GOLD 212 GB, TIMICA NU-ANTIQUE COPPER 340 AB, TIMICA NU ANTIQUE BRONZE 240 AB, CLOISONNE NU ANTIQUE GREEN 828 CB, CLOISONNE NU ANTIQUE BLUE 626 CB, GEMTONE MOONSTONE G 004, CLOISONNE NU ANTIQUE RED 424 CB, CHROMA-LITE BLACK (4498), CLOISONNE NU ANTIQUE ROUGE FLAMBE (code 440 XB), CLOISONNE NU ANTIQUE BRONZE (240 XB), CLOISONNE NU ANTIQUE GOLD (222 CB) et CLOISONNE NU ANTIQUE COPPER (340 XB) de la société ENGELHARD.

A titre encore d'exemple de pigment magnétique susceptible d'entrer dans la formulation de la composition, on peut citer les particules d'oxyde de fer noir, par exemple celles commercialisées sous la dénomination SICOVIT noir E172 par la société BASF.

Les pigments magnétiques peuvent encore comporter du fer métal, notamment du fer doux passivé, par exemple obtenu à partir de fer carbonyle en mettant en oeuvre le procédé décrit dans le brevet US 6 589 331, dont le contenu est incorporé par référence. Ces particules peuvent comporter une couche d'un oxyde de surface.

Les corps magnétiques peuvent présenter une forme plaquettaire.

La taille des corps magnétiques peut être inférieure ou égale à 10 µm, voire 1 µm.

La taille des corps magnétiques peut encore être comprise entre 30 et 80 µm, ce qui peut permettre d'obtenir un effet de pixellisation variable sous l'effet du champ magnétique, lorsque le pigment interférentiel rouge présente une taille du même ordre.

### Fibres magnétiques

Le terme « fibres » désigne des corps généralement allongés, présentant par exemple un facteur de forme allant de 3,5 à 2 500 ou de 5 à 500, par exemple de 5 à 150. Le facteur de forme est défini par le rapport L/D, où L est la longueur de la fibre et D le diamètre du cercle dans lequel s'inscrit la plus grande section transversale de la fibre.

La section transversale des fibres peut s'inscrire par exemple dans un cercle de diamètre allant de 2 nm à 500 µm, par exemple allant de 100 nm à 100 µm, voire de 1 µm à 50 µm.

Les fibres peuvent présenter par exemple une longueur allant de 1 µm à 10 mm, par exemple de 0,1 mm à 5 mm, voire de 0,3 mm à 3,5 mm.

Les fibres peuvent présenter une masse allant par exemple de 0,15 à 30 deniers (masse en gramme pour 9 km de fil), par exemple de 0,18 à 18 deniers.

La forme en section transversale des fibres peut être quelconque, par exemple circulaire ou polygonale, notamment carrée, hexagonale ou octogonale.

La composition peut comporter des fibres pleines ou creuses, indépendantes ou liées entre elles, par exemple tressées.

La composition peut comporter des fibres ayant des extrémités épointées et/ou arrondies, par exemple par polissage.

Les fibres peuvent ne pas voir leur forme sensiblement modifiée lorsqu'elles sont introduites dans la composition, étant par exemple initialement rectilignes et suffisamment rigides pour conserver leur forme. En variante, les fibres peuvent présenter une souplesse leur permettant de se déformer sensiblement au sein de la composition.

Les fibres peuvent comporter une teneur non nulle, pouvant aller jusqu'à 100 %, d'un matériau magnétique choisi parmi les matériaux magnétiques doux, les matériaux magnétiques durs, notamment à base de fer, de zinc, de nickel, de cobalt ou de manganèse et leurs alliages et oxydes, notamment Fe₃O₄, les terres rares, le sulfate de baryum, les alliages de fer silicium, éventuellement chargés en molybdène, Cu₂MnAl, MnBi, ou un mélange de ceux-ci, cette liste n'étant pas limitative.

Lorsque la composition comporte des fibres contenant des particules magnétiques, ces dernières peuvent être présentes par exemple au moins à la surface de la fibre, voire à la surface des fibres uniquement, à l'intérieur de la fibre uniquement ou encore être dispersées au sein de la fibre de manière sensiblement homogène.

Les fibres peuvent comporter par exemple un coeur non magnétique avec une pluralité de particules magnétiques à sa surface.

Les fibres peuvent encore comporter une matrice synthétique contenant une pluralité de grains magnétiques dispersés en son sein.

Le cas échéant, une matière synthétique chargée de particules magnétiques peut elle-même être enrobée par une écorce non magnétique. Une telle écorce constitue par exemple une barrière isolant le ou les matériaux magnétiques du milieu ambiant et/ou peut amener de la couleur. Les fibres peuvent comporter un coeur magnétique monolithique et être enrobées par une écorce non magnétique, ou cela peut être l'inverse.

La composition peut comporter des fibres réalisées par extrusion ou co-extrusion d'une ou plusieurs matières polymériques, notamment thermoplastiques et/ou élastomères. L'une des matières extrudées peut contenir une charge de particules magnétiques dispersées.

La fibre peut comporter une matière synthétique choisie parmi les polyamides, PET, acétates, polyoléfines, notamment PE ou PP, PVC, polyester bloc amide, Rilsan^{®} plastifié, élastomères, notamment élastomères de polyester, élastomères de PE, élastomères de silicone, élastomères de nitrile ou un mélange de ces matériaux, cette liste n'étant pas limitative.

La composition peut contenir des fibres composites comportant un coeur magnétique enrobé au moins partiellement par au moins un matériau amagnétique, synthétique ou naturel. L'enrobage du coeur magnétique peut se faire par exemple par co-extrusion, autour du coeur, d'une écorce en un matériau non magnétique.

L'enrobage du coeur peut encore s'effectuer autrement, par exemple par polymérisation *in situ.*

Le coeur peut être monolithique ou comporter une charge de grains magnétiques dispersés dans une matrice.

La composition peut encore contenir des fibres composites obtenues par enrobage par une matière synthétique, chargée de particules magnétiques, d'un coeur amagnétique, synthétique ou naturel, le coeur étant composé par exemple d'une fibre de bois, de rayonne, de polyamide, d'une matière végétale, de polyoléfine, notamment de polyéthylène, de Nylon^{®}, de polyimide-amide, d'aramide, cette liste n'étant pas limitative.

La composition peut encore comporter des particules composites magnétiques, notamment un latex magnétique.

### Particules composites magnétiques

Une particule composite magnétique est un matériau composite constitué d'une matrice organique ou minérale et de grains magnétiques. Les particules composites magnétiques peuvent ainsi comporter à leur surface et/ou en leur sein des grains d'un matériau magnétique. Les particules composites peuvent être constituées d'un coeur magnétique enrobé d'une matrice organique ou minérale, ou inversement.

Les particules composites magnétiques comportent par exemple l'un des matériaux magnétiques précités.

La dimension des particules composites magnétiques est par exemple comprise entre 1 nm et 1 mm, mieux entre 100 nm et 500 µm, mieux encore entre 500 nm et 100 µm. Par « dimension », on désigne la dimension donnée par la distribution granulométrique statistique à la moitié de la population, dite D50.

La thèse de C. GOUBAULT, 23 Mars 2004, incorporée ici par référence, rappelle au chapitre 1 l'état de l'art en matière de particules composites magnétiques, et dresse une liste de procédés de préparation pouvant être utilisés pour préparer des particules composites magnétiques, à savoir une synthèse séparée des grains magnétiques et de la matrice, une synthèse des grains magnétiques au contact de la matrice ou une synthèse de la matrice en présence des grains magnétiques.

La société KISKER commercialise des particules magnétiques composites à matrice minérale, composée de silice. Les sociétés DYNAL, SERADYN, ESTAPOR et ADEMTECH proposent des particules magnétiques composites à matrice organique, susceptibles également d'être utilisées dans l'invention.

Plus particulièrement, la société ESTAPOR commercialise sous la référence M1-070/60 des latex magnétiques constitués de grains de ferrite uniformément répartis dans une matrice polystyrène, ce latex comportant 65 % d'oxyde de fer, le diamètre moyen des particules de polystyrène étant de 890 nm et la teneur massique en matières sèches de 10 %.

### Ferrofluide

La composition **P** peut comporter un ferrofluide, c'est-à-dire une suspension colloïdale stable de particules magnétiques, notamment de nanoparticules magnétiques.

Les particules, d'une taille par exemple de l'ordre de quelques dizaines de nanomètres, sont dispersées dans un solvant (eau, huile, solvant organique), soit à l'aide d'un tensioactif ou d'un agent dispersant, soit par des interactions électrostatiques.

Les ferrofluides sont par exemple préparés par broyage de ferrites ou autres particules magnétiques jusqu'à l'obtention de nanoparticules qui sont ensuite dispersées dans un fluide contenant un surfactant, lequel s'adsorbe sur les particules et les stabilise, ou par précipitation en milieu basique d'une solution d'ions métalliques.

Chaque particule du ferrofluide présente un moment magnétique déterminé par la taille de la particule et par la nature du matériau magnétique.

Sous l'action d'un champ magnétique, les moments magnétiques des particules tendent à s'aligner suivant les lignes de champ, avec apparition d'une aimantation non nulle dans le liquide. Si le champ est annulé, il n'y a pas d'hystérésis et l'aimantation s'annule.

Au-delà d'une valeur seuil de champ, on peut également provoquer des changements macroscopiques dans le liquide, par exemple l'apparition de pics ou une modification des propriétés rhéologiques.

La dénomination « ferrofluide » englobe également une émulsion de gouttelettes de ferrofluide dans un solvant. Chaque goutte contient alors des particules magnétiques colloïdales en suspension stable. Cela permet de disposer d'un ferrofluide dans tout type de solvant. La dimension des particules magnétiques en suspension dans le ferrofluide est par exemple comprise entre 1 nm et 10 µm, mieux entre 1 nm et 1 µm, mieux encore entre 1 nm et 100 nm. Par « dimension », on désigne la dimension donnée par la distribution granulométrique statistique à la moitié de la population, dite D50.

On peut citer notamment les ferrofluides commercialisés par la société LIQUIDS RESEARCH LTD sous les références :
- WHKS1S9 (A, B ou C), qui est un ferrofluide à base aqueuse comportant de la magnétite (Fe₃O₄), ayant des particules de 10 nm de diamètre.
- WHJS1 (A, B ou C), qui est un ferrofluide à base d'iso-paraffine et de particules de magnétite (Fe₃O₄) de 10 nm de diamètre.
- BKS25_dextran, qui est un ferrofluide à base aqueuse stabilisé par du dextran, comportant des particules de magnétite (Fe₃O₄) de 9 nm de diamètre.

### Chaînes de particules et/ou de fibres magnétiques

La composition peut comporter des agglomérats de particules ou fibres dont la plus grande dimension, par exemple la longueur, est par exemple comprise entre 1 nm et 10 mm, par exemple entre 10 nm et 5 mm, ou entre 100 nm et 1 mm, ou encore entre 0,5 µm et 3,5 mm, par exemple entre 1 µm et 150 µm.

Des chaînes de particules magnétiques peuvent être obtenues par exemple en assemblant des particules magnétiques colloïdales, comme cela est décrit dans les publications « Permanently linked monodisperse paramagnetic chains », E.M. Furst, C. Suzuki, M. Fermigier, A.P. Gast, Langmuir, 14, 7334-7336 (1998), « Suspensions de particules magnétiques », M. Fermigier, Y. Grasselli, Bulletin de la SFP (105) juillet 96, et « Flexible magnetic filaments as micromechanical sensors », C. Goubault, P. Jop, M. Fermigier, J. Baudry, E. Bertrand, J. Bibette, Phys. Rev. Lett., 91, 26, 260802-1 à 260802-4 (2003), dont les contenus sont incorporés par référence.

Il est notamment décrit dans ces articles comment procéder pour obtenir des chaînes de particules de latex magnétiques comportant une matrice de polystyrène contenant des grains d'oxyde de fer et fonctionnalisées en surface, liées entre elles de façon permanente suite à une réaction chimique, notamment des liaisons covalentes entre les surfaces des particules adjacentes ; il est également décrit un procédé d'obtention de chaînes de gouttelettes d'émulsion de ferrofluides, liées entre elles par interactions de nature physique. La longueur ainsi que le diamètre des chaînes permanentes ainsi obtenues peuvent être contrôlés. De telles chaînes magnétiques constituent des objets magnétiques anisotropes orientables et déplaçables sous l'effet d'un champ magnétique.

Les dimensions des chaînes magnétiques peuvent répondre aux mêmes conditions que les fibres magnétiques.

### Agent de coloration Xchromes

Comme mentionné plus haut, l'agent de coloration Xchrome peut être choisi de manière à prendre au moins un état dans lequel il génère une couleur rouge ou proche de celle produite par interférences par le pigment interférentiel rouge ou au contraire une couleur différente.

Par « couleur proche », il faut comprendre que la longueur d'onde dominante est sensiblement la même, étant comprise entre 580 nm et 650 nm, mesurée avec le colorimètre imageant précité.

L'agent de coloration Xchrome peut encore être choisi de manière à prendre dans un état une couleur proche de celle générée par absorption par la couche de surface du pigment interférentiel. Cela peut permettre de noyer le pigment interférentiel dans la couleur du fond afin d'attirer ensuite l'attention de l'observateur sur les points de surbrillance rouge lorsque l'état de l'agent de coloration change.

Il peut s'agir d'agents de coloration photochromes.

### Agents de coloration photochromes

D'une manière générale, un agent de coloration photochrome est un agent colorant ayant la propriété de changer de teinte lorsqu'il est éclairé ou non par une lumière ultraviolette et de rétablir sa couleur initiale lorsqu'il n'est plus éclairé ou non par une lumière ou encore de passer d'un état non coloré à un état coloré et inversement. En d'autres termes, un tel agent présente des teintes différentes selon qu'il est éclairé par de la lumière contenant une certaine quantité de rayonnement UV.

L'agent de coloration photochrome peut prendre en présence d'un faible éclairage un état sensiblement non coloré, de sorte que l'intensité des points de surbrillance rouge n'est pas atténuée outre mesure par l'agent de coloration photochrome.

En présence d'un fort éclairage, l'agent de coloration photochrome peut prendre un état coloré, par exemple une teinte foncée ou une couleur rouge, atténuant l'intensité des points de surbrillance rouge, lesquels peuvent ainsi paraître moins brillants qu'en présence d'un éclairage faible. Cet effet peut surprendre l'observateur et rendre le maquillage particulièrement attractif.

L'agent de coloration photochrome peut présenter un écart ΔE au moins égal à 5. ΔE désigne l'écart de teinte observé au niveau de la matière photochromique entre son état excité, c'est-à-dire en présence d'irradiation UV et son état non excité, c'est-à-dire en absence d'irradiation UV.

On pourra utilement se référer aux exemples d'agents photochromes décrits dans US 2004/0228818, dont le contenu est incorporé par référence, notamment ceux présentant un ΔE supérieur à 5, conformément au test présenté dans ce document.

Comme exemples d'agents de coloration photochromes, figurent les dérivés naphtopyrane de type 2H-naphto-[2,1-b]-pyrane de formule (I) ou 3H-naphto-[2,1-b]-pyrane, de formule (II) : 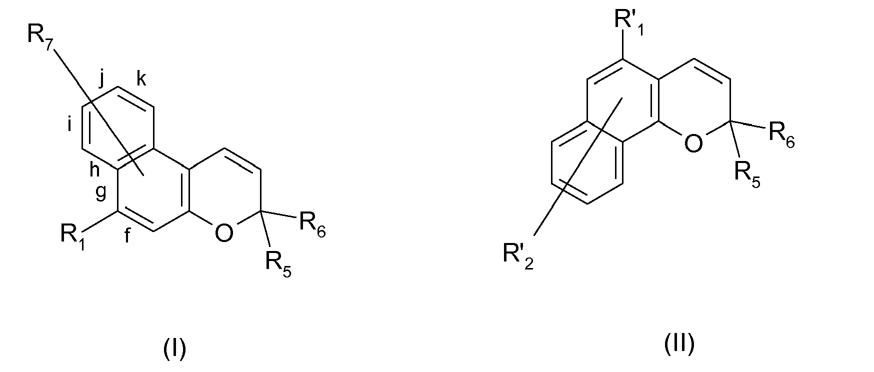 dans lesquels :
R₁ représente :
   - (i) un atome d'hydrogène;
   - (ii) un groupement hydrocarboné ayant 1 à 30 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné;
   - (iii) un cycle hydrocarboné formé avec l'une des liaisons "f" ou "gh" et le radical R₇; ou
   - (iv) un groupement choisi parmi -COOR₄, -C(O)NR₂R₃, -NR₂R₃, -OR₄ et -SR₄, dans lequel :
- R₂ et R₃ soit représentent, indépendamment l'un de l'autre, un groupement hydrocarboné ayant 1 à 20 atomes de carbone, linéaire, ramifié ou cyclique, saturé ou insaturé, éventuellement comportant 1 à 5 hétéroatomes choisis parmi N, O, S, Si et P,
   soit pris ensemble avec l'atome d'azote auquel ils sont reliés, forment un hétérocycle hydrocarboné, saturé ou insaturé, comportant 3 à 10 atomes de carbone et éventuellement 1 à 5 autres hétéroatomes choisis parmi N, O, S, Si et P, ledit cycle étant éventuellement substitué par au moins un radical hydrocarboné ayant 1 à 20 atomes de carbone, linéaire, ramifié ou cyclique, saturé ou insaturé, comportant éventuellement 1 à 5 hétéroatomes choisis parmi N, O, S, Si et P;
R₄ représente un groupement hydrocarboné ayant 1 à 20 atomes de carbone, linéaire, ramifié ou cyclique, saturé ou insaturé, éventuellement halogéné ou perhalogéné, et/ou éventuellement comportant 1 à 5 hétéroatomes choisis parmi N, O, S, Si et P;
R₅ et R₆ représentent, indépendamment l'un de l'autre, un groupement choisi parmi :
   - (i) les groupements aminoaryles cycliques saturés de formule (IIA) ou (IIB) : 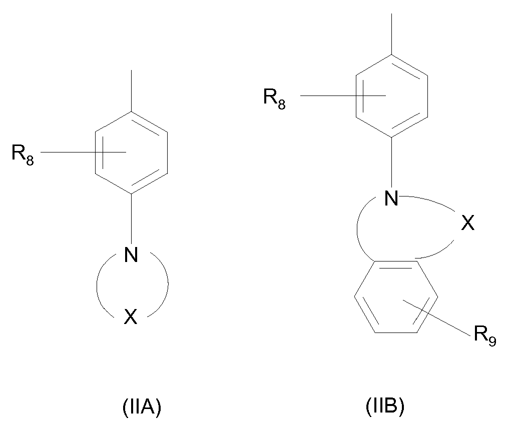 dans lesquelles le cycle comportant N et X est un cycle saturé qui comprend au total 3 à 30 atomes inclus l'azote, le reste étant des atomes de carbone et/ou des hétéroatomes choisis parmi O, S, Si, P et/ou des groupements choisis parmi -NH et -NR avec R représentant un radical hydrocarboné ayant 1 à 20 atomes de carbone, linéaire, ramifié ou cyclique, saturé ou insaturé, éventuellement comportant 1 à 5 hétéroatomes choisis parmi N, O, S, Si et P;
   - (ii) les groupements indolinoaryles de formule (III) : 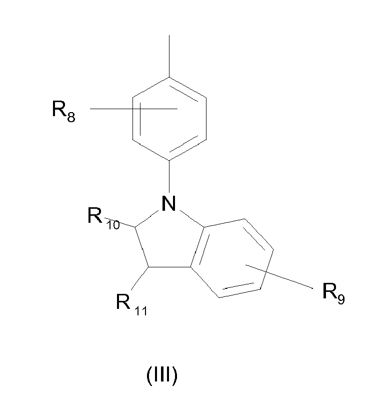 dans laquelle R₁₀ et R₁₁ représentent, indépendamment l'un de l'autre, un groupement choisi parmi (i) les groupements hydrocarbonés ayant 1 à 30 atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné ; (ii) les atomes d'halogène ; (iii) les groupements -CN (nitrile), -COOH (carboxylate), -NO₂ (nitro); (iv) un atome d'hydrogène; (v) un groupement choisi parmi -C(O)NR₂R₃, -NR₂R₃, -OR₄ ou -SR₄ avec R₂, R₃ et R₄ ayant les significations données ci-dessus; (vi) les radicaux R₁₀ et R₁₁ pouvant former ensemble un cycle hydrocarboné saturé ou insaturé ayant au total 5 à 8 atomes (incluant les atomes du cycle indoline), lesdits atomes étant choisis parmi C, O, S et/ou NR avec R représentant H ou un radical hydrocarboné ayant 1 à 20, atomes de carbone, linéaire, ramifié ou cyclique, saturé ou insaturé, éventuellement comportant 1 à 5 hétéroatomes choisis parmi N, O, S, Si et P,
   - (iii) les groupements de formule (IV) : 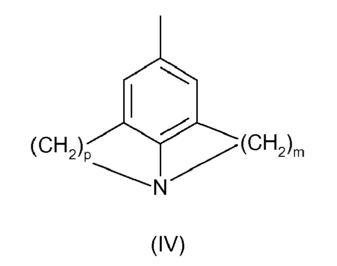 dans laquelle m et p sont, indépendamment l'un de l'autre, des entiers allant de 2à5;
   - (iv) les groupements aminoaryles cycliques insaturés de formules (VA), (VB) ou (VC) : 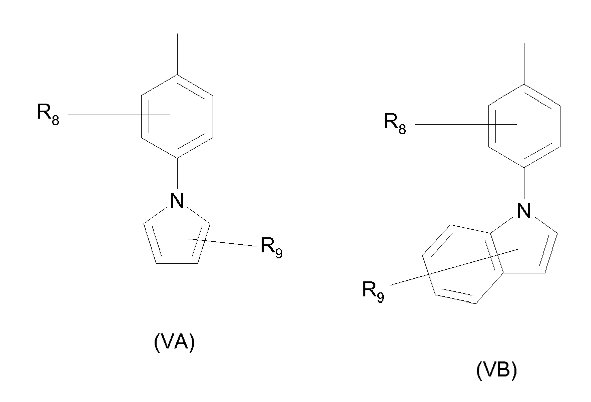 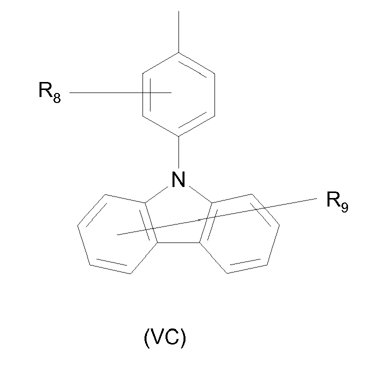 dans lesquelles R₈ et R₉, représentent, indépendamment l'un de l'autre, un groupement choisi parmi (i) les groupements hydrocarbonés ayant 1 à 30 atomes de carbone, linéaires, ramifiés ou cycliques, saturés ou insaturés, éventuellement comportant 1 à 5 hétéroatomes choisis parmi N, O, S, Si et P, et/ou éventuellement halogéné ou perhalogéné ; (ii) les atomes d'halogène ; (iii) les groupements -CN (nitrile), -COOH (carboxylate), -NO2 (nitro); (iv) un atome d'hydrogène; (v) un groupement choisi parmi -C(O)NR₂R₃, -NR₂R₃, -OR₄ ou -SR₄ avec R₂, R₃ et R₄ ayant les significations données ci-dessus;
   - (v) un groupement hydrocarboné ayant 1 à 30 atomes de carbone, linéaire, ramifié ou cyclique, saturé ou insaturé, éventuellement comportant 1 à 5 hétéroatomes choisis parmi N, O, S, Si et P; et notamment un groupement choisi parmi CONR₂R₃, -C₆H4-NR₂R₃ et -C₆H₄-OR₄ avec R₂, R₃ et R₄ ayant les significations données ci-dessus;
R₇ représente un groupement choisi parmi :
   - (i) les groupements hydrocarbonés ayant 1 à 30 atomes de carbone, linéaires, ramifiés ou cycliques, saturés ou insaturés, éventuellement comportant 1 à 5 hétéroatomes choisis parmi N, O, S, Si et P, et/ou éventuellement halogéné ou perhalogéné ;
   - (ii) les atomes d'halogène ;
   - (iii) les groupements -CN (nitrile), -COOH (carboxylate), -NO2 (nitro); -N=N- (azo); =NH (imino); -CONH₂ (amide);
   - (iv) un atome d'hydrogène;
   - (v) un groupement choisi parmi -C(O)NR₂R₃, -NR₂R₃, -OR₄ ou -SR₄ avec R₂, R₃ et R₄ ayant les significations données ci-dessus;
   - (vi) le radical R₇ pouvant en outre former, avec l'une des liaisons "i", "j", "k", ou "g,h" prises avec le radical R₁, ou "f" prise avec le radical R1, un cycle hydrocarboné saturé ayant au total 3 à 8 atomes de carbone, éventuellement comportant 1 à 5 hétéroatomes choisis parmi N, O, S, Si et P;

R'1 représente un groupement choisi parmi :
   - (i) un atome d'hydrogène;
   - (ii) un groupement hydrocarboné ayant 1 à 30 atomes de carbone, linéaires, ramifiés ou cycliques, saturés ou insaturés, éventuellement comportant 1 à 5 hétéroatomes choisis parmi N, O, S, Si et P, et/ou éventuellement halogéné ou perhalogéné ;
   - (iii) un groupement choisi parmi -C(O)NR₂R₃, -NR₂R₃, -OR₄ et -SR₄, avec R₂, R₃ et R₄ ayant les significations données ci-dessus;
R'₂ représente un groupement choisi parmi :
   - (i) les groupements hydrocarbonés ayant 1 à 30 atomes de carbone, linéaires, ramifiés ou cycliques, saturés ou insaturés, éventuellement comportant 1 à 5 hétéroatomes choisis parmi N, O, S, Si et P, et/ou éventuellement halogéné ou perhalogéné ;
   - (ii) les atomes d'halogène ;
   - (iii) les groupements -CN (nitrile), -COOH (carboxylate), -NO₂ (nitro); -N=N- (azo); =NH (imino); -CONH₂ (amide);
   - (iv) un atome d'hydrogène;
   - (v) un groupement choisi parmi -C(O)NR₂R₃, -NR₂R₃, -OR₄ ou -SR₄ avec R₂, R₃ et R₄ ayant les significations données ci-dessus.

Comme exemples d'agents photochromes, on peut encore citer le diaryléthène, de formule 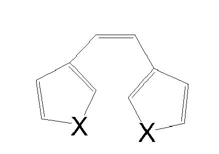 et ses dérivés,
le dihydroazulène/vinylhepta fulvène, de formule 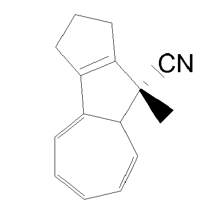 et ses dérivés,
le spyronaphtoxazine, de formule 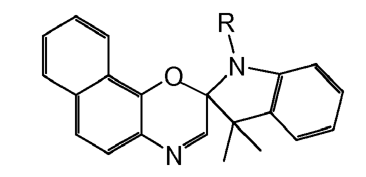 et ses dérivés.

L'agent photochrome peut être un composé organique ou inorganique. Lorsque l'agent photochrome est un composé organique, le changement de couleur peut généralement être plus rapide et intense.

A titre d'exemples d'agents photochromes, on peut citer le Photosol® de la société PPG, qui change de manière réversible de couleur lorsqu'activé par un rayonnement UV de longueur d'onde comprise entre 300 et 360 nm, le Reversacol® de la société J. ROBINSON et le Photogenica® de la société CATALYST & CHEMICALS.

### Agents thermochromes

Un agent thermochrome est un pigment ou colorant qui peut changer de couleur en fonction de la température.

L'agent thermochrome présente par exemple une couleur qui est perdue lorsque la température dépasse une certaine valeur, par exemple 15 °C environ ou 30 °C environ, selon la nature de l'agent thermochrome.

L'agent thermochrome peut comporter des capsules d'un polymère contenant un solvant, ce solvant permettant, selon son étant fondu ou non, à des composés d'entrer en contact et de modifier des propriétés d'absorption du rayonnement lumineux.

Le changement de couleur peut être réversible.

On peut par exemple utiliser l'agent thermochrome commercialisé sous la référence Kromafast® Yellow5GX 02 par la société KROMACHEM LTD, ou encore Chromazone® en poudre ou dispersion, Thermobatch® ou Thermostar®, de la société CHROMAZONE.

### Agents piézochromes et tribochromes

Un agent piézochrome est susceptible de changer de couleur en présence d'une force mécanique.

Comme exemples d'agents piézochromes, on peut citer le diphenylflavylene.

Un agent tribochrome est susceptible de changer de couleur en présence d'une force mécanique, de manière plus durable que dans le cas des agents piézochromes.

On pourra se référer à la publication WO 94/26729, dont le contenu est incorporé par référence.

### Agents mécanoluminescents

Ces agents sont capables d'émettre une lumière lorsqu'ils reçoivent une sollicitation mécanique telle qu'une compression, un cisaillement ou un frottement.

L'agent mécanoluminescent est de préférence sous forme de particule insoluble dans le milieu cosmétique. La taille moyenne des particules est par exemple entre 0,01 et 50 µm, de préférence entre 0,1 et 10 µm.

Comme matériaux mécano luminescents, on peut citer :
a) les complexes et chélates de lanthanides tels que ceux décrits dans les publications US 6 071 632, US 2002/0015965 et WO 09/016429 dont les contenus sont incorporés par référence. Les terres rares sont de préférence choisies parmi l'europium, le terbium, le samarium et le dysprosium. Dans ces matériaux, des dicétones sont utilisées comme ligand des sels de lanthanide trivalent. Ces matériaux en milieu organique [?].
b) Les aluminates, silicates et aluminosilicates dopés avec des ions de terres rares tels que décrits dans les publications US 6 280 655, EP 1 318 184, JP 2002/194349, JP 2004/59746, dont les contenus sont incorporés par référence, notamment (Sr,Mg,Ba,Zn,Ca) Al₂O₄,(SrLa,SrY)Al₃O₇(Sr₂,SrMg,SrCa,SrBa)Al₆O₁₁, Sr₂(Mg,A1)(A1,Si)SiO₇, Sr(Zn,Mn,Fe,Mg)Si₂O₆. Les éléments indiqués entre parenthèses sont partiellement ou entièrement interchangeables. Des ions de terres rares tels que le cérium, l'europium, le samarium, le néodymium, le gadolinium, le disprosium et le terbium peuvent être utilisés, seuls ou en mélange. L'europium et le disprosium sont préférés.
c) Le sulfure de zinc, le sulfure de manganèse, le sulfure de cuivre, le sulfure de cadmium ou l'oxyde de zinc, éventuellement dopés avec des ions de métaux de transition ou des ions de terres rares comme décrit dans les publications US 6 117 574 et JP 2004/43656 dont les contenus sont incorporés par référence. Les ions de métaux de transition sont de préférence le cuivre ou le manganèse. Les ions de terres rares sont de préférence l'europium ou le cérium. Parmi ces matériaux, ZnS : Mn est préféré.

Les matériaux listés sous b) et c) peuvent être synthétisés par une réaction en phase solide impliquant un mélange à sec suivi d'un traitement thermique et de frittage à haute température ou par un process sol-gel suivi d'un séchage, chauffage et frittage. La température de frittage est par exemple supérieure à 1 000 °C.

Les matériaux listés sous b) sont préférés. Parmi ceux-ci, SrAl₂O₄ et SrMgAl₁₀O₁₇ dopés avec des métaux rares sont préférés.

Des pigments mécano luminescents SrA12O4 dopés avec des ions de métaux rares sont vendus sous la référence TAIKO-M1-1 par la société TAIKO Refractories Co., Ltd. Les particules de ce pigment ont un diamètre compris entre 5 et 10 µm et une luminescence verte sous une faible contrainte mécanique.

### Agents solvatochromes

Un agent solvatochrome est susceptible de changer de couleur en présence de solvants. Le colorant DC Red27 en est un exemple, ce composé présentant dans une formulation anhydre une absence de couleur et l'adjonction d'eau révélant une couleur rose.

### Formes galéniques

La composition cosmétique peut se présenter sous toute forme galénique normalement utilisée pour une application topique et notamment sous forme anhydre, sous forme solide ou semi-solide, sous forme d'une solution huileuse ou aqueuse, d'un gel huileux ou aqueux, d'une émulsion huile-dans-eau ou eau-dans-huile, d'une émulsion multiple, d'une dispersion d'huile dans de l'eau grâce à des vésicules situés à l'interface huile/eau, d'un spray, à condition de conserver les points de surbrillance rouge.

La composition peut constituer tout produit destiné à être appliqué sur la peau, par exemple un fond de teint, un produit de contour des yeux, un blush, une ombre à paupières, une base de maquillage, un produit de maquillage du corps.

La composition de l'invention peut être obtenue selon les procédés de préparation classiquement utilisés en cosmétique.

### Conditionnement et modes d'application

La composition peut être conditionnée dans tout réceptacle ou sur tout support prévu à cet effet.

La composition selon l'invention peut être sous forme de solide, notamment de pain ou raisin ou de poudre, de semi-solide, de liquide, de pâte ou de crème plus ou moins fluide.

La composition peut être appliquée en utilisant un applicateur, floqué ou non, par exemple une mousse, un embout, un pinceau, un feutre, une spatule, un fritté, une brosse, un peigne, un tissé ou non tissé.

L'application peut encore s'effectuer avec le doigt ou en déposant directement la composition sur le support à maquiller, par exemple par friction ou pulvérisation ou projection à l'aide d'un dispositif piézoélectrique ou par transfert d'une couche de composition préalablement déposée sur un support intermédiaire.

La composition peut être appliquée par exemple selon une épaisseur comprise par exemple entre 1 et 10 µm.

L'application de la composition s'effectue par exemple avec une densité massique comprise entre 1 et 5 mg/cm².

La composition peut, le cas échéant, être appliquée comme couche de base (*base coat*) recouverte d'une couche d'une autre composition (*top coat*) ou comme couche de revêtement sur une couche d'une autre composition, voire entre une couche de base et une couche de revêtement afin par exemple d'accroître la tenue et/ou la brillance.

### Dispositifs magnétiques

Selon un autre de ses aspects, l'invention a encore pour objet un kit comportant une composition telle que définie ci-dessus et au moins un dispositif magnétique permettant de générer un champ magnétique permettant de déplacer et/ou modifier l'orientation des corps magnétiques.

Le dispositif magnétique peut comporter un aimant permanent ou un électroaimant, alimenté par exemple par au moins une pile ou un accumulateur. Dans ce dernier cas, le dispositif magnétique peut comporter un interrupteur permettant d'alimenter sélectivement l'électroaimant en électricité.

Le dispositif magnétique peut être agencé pour créer un champ magnétique dont l'orientation varie dans le temps. Lorsque le dispositif magnétique comporte un aimant, le dispositif peut par exemple comporter un moteur permettant d'entraîner en rotation l'aimant. En variante, le dispositif magnétique peut comporter plusieurs solénoïdes disposés de manière à générer, lorsqu'alimentés séquentiellement en électricité, un champ magnétique tournant.

Un champ magnétique rotatif peut permettre par exemple d'obtenir un motif présentant une symétrie de révolution, par exemple un motif donnant l'impression d'une sphère en relief.

Le ou les électroaimants peuvent être alimentés en permanence ou par intermittence, au choix de l'utilisateur. En particulier, le dispositif magnétique peut être agencé de telle sorte que le ou les électroaimants puissent ne pas être alimentés tant que le dispositif magnétique n'est pas positionné correctement près du support revêtu de la première composition.

Le champ magnétique est par exemple d'au moins 50 mT, voire d'au moins 0,2 T, voire d'au moins 1 T (10 000 Gauss).

De manière à rendre plus facile l'application du champ magnétique, le dispositif magnétique peut comporter un organe permettant de le positionner relativement au support sur lequel la composition a été déposée. Cela peut permettre par exemple d'éviter que le dispositif magnétique ne vienne accidentellement en contact avec la composition et/ou de centrer le motif réalisé sur la région concernée.

Dans un exemple de mise en oeuvre de l'invention, le dispositif magnétique est solidaire d'un applicateur servant à l'application de la composition cosmétique. Cela peut permettre de réduire le nombre d'objets manipulés par l'utilisateur et faciliter le maquillage.

Dans un autre exemple de mise en oeuvre de l'invention, le dispositif magnétique comporte un aimant monté à une première extrémité d'une tige dont la deuxième extrémité est reliée à un organe de préhension d'un applicateur servant à l'application de la composition cosmétique.

Le champ magnétique peut encore être exercé au moyen d'une structure magnétique, notamment souple, comportant une alternance de pôles N et S. Une telle structure peut permettre par exemple de réaliser des motifs répétitifs sur la composition, par exemple des rayures.

### Procédé de maquillage

Selon encore un autre de ses aspects, l'invention a pour objet un procédé de maquillage consistant à appliquer par l'intermédiaire d'au moins une composition cosmétique, sur les matières kératiniques, au moins un premier pigment interférentiel apte à générer, lorsque la composition est appliquée sur un support, des points de surbrillance de couleur rouge d'intensité supérieure ou égale à 3 000 cd m⁻² et de longueur d'onde dominante comprise entre 580 et 650 nm et des particules réfléchissantes aptes à générer sur ledit support d'autres points de surbrillance, d'intensité de brillance supérieure ou égale à celle du pigment interférentiel rouge.

Le premier pigment interférentiel et les particules réfléchissantes peuvent être appliqués par l'intermédiaire de la même composition.

Le premier pigment interférentiel et les particules réfléchissantes peuvent encore être appliqués *via* deux compositions différentes contenant respectivement le pigment interférentiel rouge et l'agent de coloration sensible à au moins un stimulus extérieur.

Selon un autre de ses aspects, l'invention a encore pour objet un procédé de maquillage des fibres kératiniques, comportant les étapes suivantes :
1) appliquer sur les matières kératiniques une couche d'une composition telle que définie ci-dessus,
2) modifier, en soumettant le dépôt à un champ magnétique, l'orientation et/ou la position d'une partie au moins des corps magnétiques au sein de la couche de la couche ainsi déposée.

La présente invention a également pour objet un procédé de maquillage consistant à appliquer, par l'intermédiaire d'au moins une composition cosmétique, sur les matières kératiniques, un pigment interférentiel de couleur rouge apte à générer des points de surbrillance d'intensité supérieure ou égale à 3000 cd.m⁻² et de longueur d'onde dominante comprise entre 580 et 650 mm et des corps magnétiques présentant une susceptibilité magnétique non nulle.

Le pigment interférentiel de couleur rouge et les corps magnétiques peuvent être appliqués par l'intermédiaire de la même composition.

Le pigment interférentiel de couleur rouge et les corps magnétiques peuvent encore être appliqués via deux compositions différentes contenant respectivement le pigment interférentiel rouge et les corps magnétiques.

Selon autre de ses aspects, l'invention a également pour objet un procédé de maquillage consistant à appliquer par l'intermédiaire d'au moins une composition cosmétique, sur les matières kératiniques, au moins un pigment interférentiel rouge apte à générer, après application, des points de surbrillance d'intensité supérieure ou égale à 3000 cd.m² et de longueur d'onde dominante comprise entre 580 et 650 nm et au moins un deuxième pigment réfléchissant, argenté ou coloré et de longueur d'onde dominante λ₂ telle que |λ₁ - λ₂| ≥ 50 nm, ce deuxième pigment ayant une taille moyenne supérieure ou égale à 30 µm, mieux 40 µm.

Le pigment interférentiel rouge et le deuxième pigment réfléchissant peuvent être appliqués par l'intermédiaire de la même composition.

Le pigment interférentiel rouge et le deuxième pigment réfléchissant peuvent encore être appliqués *via* deux compositions différentes contenant respectivement le pigment interférentiel rouge et le deuxième pigment réfléchissant.Selon un autre de ses aspects, l'invention a également pour objet un procédé de maquillage consistant à appliquer,par l'intermédiaire d'au moins une composition cosmétique, sur les matières kératiniques, au moins un pigment interférentiel rouge apte à générer, après application, des points de surbrillance d'intensité supérieure ou égale à 3000 cd.m⁻² et de longueur d'onde dominante comprise entre 580 et 650 nm et au moins un agent de coloration sensible à au moins un stimulus extérieur.

Le pigment interférentiel rouge et l'agent de coloration sensible à au moins un stimulus extérieur peuvent être appliqués par l'intermédiaire de la même composition.

Le pigment interférentiel rouge et l'agent de coloration sensible à au moins un stimulus extérieur peuvent encore être appliqués via deux compositions différentes contenant respectivement le pigment interférentiel rouge et l'agent de coloration sensible à au moins un stimulus extérieur.

### Kit

Selon un autre de ses aspects, l'invention a également pour objet un kit de maquillage comportant :
- une première composition comportant, dans un milieu cosmétiquement acceptable, au moins un premier pigment interférentiel apte à générer, lorsque la composition est appliquée sur un support, des points de surbrillance de couleur rouge d'intensité supérieure ou égale à 3 000 cd m⁻² et de longueur d'onde dominante comprise entre 580 et 650 nm,
une deuxième composition comportant, dans un milieu cosmétiquement acceptable, des particules réfléchissantes aptes à générer sur ledit support d'autres points de surbrillance, d'intensité de brillance supérieure ou égale à celle du pigment interférentiel rouge.

Selon un autre de ses aspects, l'invention concerne également un kit de maquillage comportant :
- une première composition comportant, dans un milieu cosmétiquement acceptable, un pigment interférentiel de couleur rouge apte à générer des points de surbrillance d'intensité supérieure ou égale à 3000 cd.m⁻² et de longueur d'onde dominante comprise entre 580 et 650 mm,
- une deuxième composition comportant, dans un milieu cosmétiquement acceptable, des corps magnétiques présentant une susceptibilité magnétique non nulle.

La deuxième composition peut être appliquée sous ou sur la première.

Selon un autre de ses aspects, l'invention a également pour objet un kit de maquillage comportant :
- une première composition comportant, dans un milieu cosmétiquement acceptable, au moins un pigment interférentiel rouge apte à générer, lorsque la composition est appliquée sur un support, des points de surbrillance rouge d'intensité supérieure ou égale à 3 000 cd.m⁻² et de longueur d'onde dominante comprise entre 580 et 650 nm,
- une deuxième composition comportant, dans un milieu cosmétiquement acceptable, au moins un deuxième pigment réfléchissant, argenté ou coloré et de longueur d'onde dominante λ₂ telle que |λ₁ - λ₂| ≥ 50 nm, ce deuxième pigment ayant une taille moyenne supérieure ou égale à 30 µm, mieux 40 µm.

Selon un autre de ses aspects, l'invention a également pour objet un kit de maquillage comportant :
- une première composition comportant, dans un milieu cosmétiquement acceptable, au moins un pigment interférentiel rouge apte à générer, lorsque la composition est appliquée sur un support, des points de surbrillance d'intensité supérieure ou égale à 3000 cd.m⁻² et de longueur d'onde dominante comprise entre 580 et 650 nm,
- une deuxième composition comportant, dans un milieu cosmétiquement acceptable, au moins un agent de coloration sensible à au moins un stimulus extérieur.

### Exemples proposés

Les teneurs indiqués sont massiques

### Exemple 1 (Blush)

| | |
|---|---|
| Sulfate de magnésium | 1,5 |
| Carboxyméthyl cellulose de sodium | 0,5 |
| Hectorite modifiée distéaryl diméthyl ammonium | 1 |
| Cyclopenta diméthylsiloxane | 16 |
| Glycérol | 5 |
| Mélange de poly-méthylcétyl diméthyl méthysiloxane | |
| oxyéthylène, isostéarate poyglycérol (4 moles) laurate | |
| d'hexyle | 9 |
| Eau | 41,6 |
| Mélange de stéarate d'éthylène glycol acétyle, | |
| tri-stéarate de glycéryle | 0,3 |
| Poly diméthylsiloxane (viscosité : 5 cst) | 16 |
| Pigments interférentiels rouge* | 5 |
| 1,5-pentanediol | 3 |

| | |
|---|---|
| * Pigment interférentiel rouge à coeur de silice enrobé d'une couche d'oxyde de fer, commercialisé par la société MERCK sous la référence XIRONA LE ROUGE. | |

Le teint est perçu comme étant plus chaud et avec plus d'éclat.

### Exemple 2 (Blush)

| | |
|---|---|
| Triéthanolamine | 1 |
| Acide éthylène diamine tétracétique, sel disodique, 2H₂O | 0,2 |
| Homopolymère carboxyvinylique réticulée | 0,5 |
| Polyvinylpyrrolidone | 0,6 |
| Glycérol | 5,75 |
| Eau désionisée | 83,05 |
| 1,3 butylène glycol | 2 |
| Microsphère de silice (3 µm) | 1,5 |
| Pigment interférentiel rouge* | 5 |

| | |
|---|---|
| * idem exemple 1. | |

L'effet est sensiblement le même qu'à l'exemple 1.

### Exemple 3 (Blush)

| | |
|---|---|
| Octyl-2 dodécanol | 10 |
| Ditertiobutyl 4-hydroxytoluene | 0,07 |
| Polybutène (monooléfines / isooaraffines 95/5) (PM : 2060) | 50 |
| Mélange de p-hydroxybenzaotes d'isopropyle, iso-butyle, n-butyle (40/30/30) | 0,4 |
| Tetra-iso-stéarate de pentaérythrityle | 11,33 |
| Tr-mellitate de tridécyle | 13 |
| Triglycéride d'acide 2-décyl tétradécanoïque (GUEBET C24) | 13 |
| Pigment magnétique* | 0,2 |
| Pigment interférentiel rouge** | 2 |

| | |
|---|---|
| * Pigment de fer doux aciculaire ** Pigment interférentiel comportant un coeur de silice enrobé d'une couche d'oxyde de fer, commercialisé par la société MERCK sous la référence XIRONA LE ROUGE. | |

### Exemple 4

### Couche de base

| | |
|---|---|
| Tri-mellitate de tri-decyle | 11 |
| Lanoline liquide | 10 |
| Malate de d'iso-stearyle | 13 |
| Lanoline acetylée | 10 |
| Triglycerides d'acides laurique/palmitique/cetylique/stearique 50/20/10/10 | 5 |
| Cire microcrystalline (C20-C60) | 3 |
| Lanolate d'isopropyle protégé | 10 |
| Octyl-2-decanol | 16 |
| Phényl trimethylsiloxy trisiloxane (VISCOSITE : 20 CST - PM : 372) | 4 |
| Cire de polyethylene (PM : 500) | 8 |
| Pigment DC RED7 CI 15850 | 5 |

### Couche de recouvrement

| | |
|---|---|
| Octyl-2 dodécanol | 10 |
| Ditertiobutyl 4-hydroxytoluene | 0,07 |
| Polybutène (monooléfines/isoparaffines 95/5) (PM : 2060) | 50 |
| Mélange de p-hydroxybenzoates d'isopropyle, iso-butyle, n-butyle (40/30/30) | 0,4 |
| Tetra-iso-stéarate de pentaérythrityle | 11,33 |
| Tri-mellitate de tridécyle | 12 |
| Triglycéride d'acide 2-décyl tétradécanoïque (GUERBET C24) | 11 |
| Pigment interférentiel coloré* | 5 |
| * Pigment interférentiel rouge comportant un coeur de silice enrobé d'une couche d'oxyde de fer Fe₂O₃ disponible auprès de la société MERCK sous la référence XIRONA LE ROUGE. | |

La couche de base forme un fond continu uniforme. On obtient grâce à la couche de recouvrement un effet chatoyant d'autant plus visible que l'agent de coloration de la couche de base est séparé du pigment interférentiel rouge.

Bien entendu, l'invention n'est pas limitée aux exemples qui viennent d'être donnés. L'expression « comportant un » est synonyme de « comportant au moins un » et « compris entre » s'entend bornes incluses.

## Revendications

1. Procédé de maquillage des peaux foncées, comportant l'étape consistant à appliquer sur la peau une composition cosmétique comportant dans un milieu cosmétiquement acceptable au moins un pigment interférentiel de couleur rouge, apte à générer, lorsque la composition est appliquée sur la peau, des points de surbrillance d'intensité supérieure ou égale à 3000 cd m⁻² et de longueur d'onde dominante comprise entre 580 nm et 650 nm.

2. Procédé selon la revendication 1, le pigment interférentiel étant apte à générer des points de surbrillance d'intensité supérieure ou égale à 3400 cd m⁻².

3. Procédé selon la revendication 1 ou 2, la composition présentant une saturation C* supérieure ou égale à 30.

4. Procédé selon la revendication 3, la saturation étant supérieure ou égale à 35.

5. Procédé selon l'une quelconque des revendications 1 à 4, la composition présentant un angle de teinte h° compris entre 10° et 30°.

6. Procédé selon la revendication 5, l'angle de teinte étant compris entre 15° et 25°.

7. Procédé selon l'une quelconque des revendications précédentes, la teneur massique en pigment interférentiel rouge allant de 0,1 % à 15 % si la composition est liquide et de 0,1 à 60 % si la composition est solide.

8. Procédé selon l'une quelconque des revendications précédentes, la composition étant dépourvue d'agents de coloration autres que le pigment interférentiel rouge.

9. Procédé selon l'une quelconque des revendications 1 à 8, la taille du pigment interférentiel rouge étant supérieure ou égale à 30 µm, voire 40 µm.

10. Procédé selon l'une quelconque des revendications 1 à 9, la taille du pigment interférentiel rouge étant comprise entre 30 µm et 80 µm.

11. Procédé selon l'une quelconque des revendications 1 à 10, le pigment interférentiel rouge comportant un coeur inorganique.

12. Procédé selon la revendication 11, le coeur étant en silice, en verre ou en mica.

13. Procédé selon l'une quelconque des revendications précédentes, comportant une couche de surface d'un oxyde métallique.

14. Procédé selon la revendication 13, l'oxyde métallique comportant de l'oxyde de fer Fe₂O₃.

15. Procédé selon l'une quelconque des revendications 1 à 14, la composition comportant, un colorant.

16. Composition de maquillage comportant, dans un milieu cosmétiquement acceptable, au moins un pigment interférentiel de couleur rouge, apte à générer, lorsque la composition est appliquée sur la peau, des points de surbrillance d'intensité supérieure ou égale à 3000 cd m⁻² et de longueur d'onde dominante comprise entre 580 nm et 650 nm et au moins un colorant.

17. Composition selon la revendication 15, le colorant ayant une longueur d'onde dominante comprise entre 580 et 650 nm.

18. Composition selon la revendication 16, le colorant étant hydrosoluble.

19. Composition selon la revendication 16, le colorant étant liposoluble.

20. Composition selon la revendication 16, le colorant étant choisi parmi le caramel, le jus de betterave et le carmin, la bétanine (betterave), la chlorophylline cuivrée, le bleu de méthylène, les anthocyanines (enocianine, carotte noire, hibiscus, sureau), la riboflavine, le rouge Soudan, le β-carotène, les caroténoïdes, le lycopène, l'huile de palme, le brun Soudan, le jaune quinoléique les xanthophylles (capsanthine, capsorubine, lutéine), le curcumin, les anthocyanes de fleurs ou de fruits ou leurs dérivés, les flavonoïdes et tanins extraits de végétaux natifs ou fermentés, la juglone, la lawsone, les extraits de soja fermenté, d'algues, de champignons, de micro-organismes, les sels de Flavylium non substitués en position 3, les extraits de Gesneria Fulgens, Blechum Procerum, Saxifraga et les pigments susceptibles d'être obtenus par extraction par un solvant organique ou hydro-organique d'un milieu de culture de micromycètes du type monascus Monascus, les colorants liposolubles synthétiques tels que le DC Red 17, le DC Red 21, le DC Red 27, le DC Green 6, le DC Yellow 11, le DC Violet 2 et le DC Orange 5, FDC Red 4, le DC Red 6, le DC Red 22, le DC Red 28, le DC Red 30, le DC Red 33, le DC Orange 4, le DC Yellow 5, le DC Yellow 6, le DC Yellow 8, le FDC Green 3, le DC Green 5 et le FDC Blue 1.

21. Composition selon l'une des revendications 16 et 17, la taille du pigment interférentiel rouge étant comprise entre 30 µm et 80 µm.

22. Composition selon la revendication 16, le pigment interférentiel rouge étant apte à créer des points de surbrillance d'intensité supérieure ou égale à 3500 cd.m⁻², la composition ne contenant pas de corps solides générant une couleur par absorption ou de charges blanches dans le milieu ou, lorsque la composition en contient, la teneur massique totale en de tels corps solides étant inférieure ou égale à 1 % par rapport au poids total de la composition.

23. Composition selon la revendication 16, comportant des particules réfléchissantes aptes à générer sur ledit support d'autres points de surbrillance, d'intensité de brillance supérieure ou égale à celle du pigment interférentiel rouge.

24. Composition selon la revendication 16, présentant un indice de turbidité inférieur ou égal à 100 NTU.

25. Composition selon la revendication 16, comportant des corps magnétiques présentant une susceptibilité non nulle.

26. Composition selon la revendication 16, comportant un deuxième pigment réfléchissant argenté ou un pigment réfléchissant coloré et de longueur d'onde dominante λ₂ telle que |λ₁ - λ₂| ≥ 50 nm, ce deuxième pigment ayant une taille moyenne supérieure ou égale à 30 µm, mieux à 40 µm.

27. Composition selon la revendication 16, comportant au moins un agent de coloration sensible à au moins un stimulus extérieur.

28. Gamme d'au moins deux compositions cosmétiques selon la revendication 16, la différence de saturation entre deux compositions de la gamme étant inférieure ou égale à 2, le pigment interférentiel rouge étant dans ces deux compositions à des concentrations différant d'au moins 1 %.
